# EUROPEAN PATENT APPLICATION

(11) **EP 3 017 706 A1**
(43) Date of publication of application: **11.05.2016**
(21) Application number: 14191889.6
(22) Date of filing: 05.11.2014
(51) Int. Cl.: A23L 7/20, A23L 7/25, C12N 9/24, C12C 1/02, C12C 5/00, C12N 9/42

(54) **Enzymes for malting**

(71) Applicant: DuPont Nutrition Biosciences ApS, 1001 Copenhagen K (DK)
(72) Inventor: Poulsen, Charlotte Horsmans, 8220 Brabrand (DK)

(57) **Abstract**

The present disclosure provides methods and compositions for preparation of malted cereals, the improved malted cereals and their use, e.g., in the production of food and beverages.

## Description

### BACKGROUND

Malting is the process of converting barley and other cereals into malt, typically, for use in brewing, distilling, or in foods. The malting process allows the grain to partially germinate, making the seed's resources available. For example, malting grains develops the enzymes required to modify the grain's starches into sugars, as well as other enzymes, such as proteases, which break down the proteins in the grain into forms that can be used by the fermenting host.

In traditional malting processes, malting process starts with drying the grains to a moisture content below 14%, and then storing for around six weeks to overcome seed dormancy. When ready, the moisture content of cereals is raised by steeping in water several times over a time period, usually several days, to allow the grain to absorb moisture and to start to sprout or germinate. When the grain has a moisture content of around 46%, it is transferred to the germination floor, where the grain is allowed to germinate. The grain at this point is called "green malt". The green malt is then kilned to the desired specification.

The process of steeping is very water consuming. Typically, to produce 1 liter of malt, the malting process consumes 5 to 10 liters of water. In recent years, there has been a dramatic change in energy and raw material prices caused by increased demand for grains, global water shortage, changing weather factors, etc. Thus, there is a need for improved processes in food production, brewing, and distilling that will bring down costs, increase production efficiency and decrease the amount of water needed.

### SUMMARY OF THE INVENTION

The present disclosure provides compositions and processes for the preparation of malted cereals.

Aspects and embodiments of the compositions and methods are set forth in the following separately numbered paragraphs.
1. A method of producing malted cereal comprising:
   a) providing a cereal grain comprising one or more β-glucans,
   b) adding to the cereal an effective amount of a β-glucanase during a malting process; and
   c) obtaining a malted cereal.
2. The method of paragraph 1, where the cereal grain further comprises one or more arabinoxylans.
3. In some embodiments of the method of paragraph 2, the method further comprises adding to the cereal an effective amount of a xylanase.
4. In some embodiments of the method of any preceding paragraphs, the method comprises a steeping step including one or more wetting stages, where the β-glucanase and/or the xylanase are added one or more times during the one or more of wetting stages.
5. In some embodiments of the method of paragraph 4, the β-glucanase and/or the xylanase are added at the beginning of the first wetting of the cereal.
6. In some embodiments of the method of paragraph 4, the β-glucanase and/or the xylanase are added during the first wetting of the cereal.
7. In some embodiments of the method of paragraph 4, the β-glucanase and/or the xylanase are added at the beginning of the second wetting of the cereal.
8. In some embodiments of the method of paragraph 4, the β-glucanase and/or the xylanase are added during the second wetting of the cereal.
9. In some embodiments of the method of paragraph 4, the β-glucanase and/or the xylanase are added at the beginning of the third wetting of the cereal.
10. In some embodiments of the method of paragraph 4, the β-glucanase and/or the xylanase are added during the third wetting of the cereal.
11. In some embodiments of the method of paragraph 4, the β-glucanase and/or the xylanase are added at the beginning of all wetting stages of the cereal.
12. In some embodiments of the method of paragraph 4, the β-glucanase and/or the xylanase are added during all wetting stages of the cereal.
13. In some embodiments of the method of paragraph 4, the one or more wetting stages comprise spraying the cereal and where the β-glucanase and/or the xylanase are added one or more times, or constantly, during the spraying.
14. In some embodiments of the method of paragraphs 4-13, the β-glucanase and/or the xylanase are added after a desired temperature has been reached.
15. In some embodiments of the method of any preceding paragraph, the cereal reaches a moisture content of between about 40 to about 50 % W/W.
16. In some embodiments of the method of paragraph 15, the moisture content is reached in less than 12 hours.
17. In some embodiments of the method of paragraph 15 or 16, the moisture content is reached with at least 10 % less water than the water used for a malted cereal prepared without the β-glucanase and/or the xylanase.
18. In some embodiments of the method of any preceding paragraph, the cereal has 1-10 % W/W of β-glucans.
19. In some embodiments of the method of any preceding paragraph, the cereal has 1-10 % W/W of arabinoxylans.
20. In some embodiments of the method of any preceding paragraph, the amount of high molecular weight β-glucans in the cereal is decreased at least 50%.
21. In some embodiments of the method of any preceding paragraph, the amount of high molecular weight β-glucans in the cereal is decreased at least 80%.
22. In some embodiments of the method of paragraphs 3-20, where of the amount of high molecular weight arabinoxylans in the cereal is decreased at least 50%.
23. In some embodiments of the method of any preceding paragraph, the cereal is selected from the group consisting of barley, wheat, rye, corn, oats, rice, millet, triticale, cassava, sorghum and a combination thereof.
24. In some embodiments of the method of any preceding paragraph, the β-glucanase is dosed at 0.01-100mg enzyme protein per kilogram of cereal.
25. In some embodiments of the method of any preceding paragraph the β-glucanase is dosed at 1-15 mg enzyme protein per kilogram of cereal.
26. In some embodiments of the method of any preceding paragraph, the xylanase is dosed at 0.01-100 mg enzyme protein per kilogram of cereal.
27. In some embodiments of the method of any preceding paragraph, the xylanase is dosed at 1-15 mg enzyme protein per kilogram of cereal.
28. In some embodiments of the method of any preceding paragraph, the β-glucanase is an endo-1,3(4)-β-glucanase.
29. In some embodiments of the method of paragraph 28, the endo-1,3(4)-β-glucanase enzyme comprises an amino acid sequence having at least 80% identity with any one selected from SEQ ID NO:7, SEQ ID NO:8, SEQ ID NO:9, SEQ ID NO:10, and SEQ ID NO:11, SEQ ID NO:12, SEQ ID NO:13, SEQ ID NO:14, SEQ ID NO:15, SEQ ID NO:16, or any functional fragment thereof
30. In some embodiments of the method of paragraphs 3-29, the xylanase is an endo-1,4-β-xylanase.
31. In some embodiments of the method of paragraph 30 the endo-1,4-β-xylanase enzyme comprises an amino acid sequence having at least 80% identity with any one selected from SEQ ID NO:1, SEQ ID NO:2, SEQ ID NO:3, SEQ ID NO:4, SEQ ID NO:5, SEQ ID NO:6, SEQ ID NO:17, and SEQ ID NO:18, or any functional fragment thereof.
32. In some embodiments of the method of paragraphs 3-31, the β-glucanase and the xylanase have an amino acid sequence having at least 80% sequence identity with the respective SEQ ID, or any functional fragment thereof, being selected from the list consisting of SEQ ID NO:1 and SEQ ID NO:7; SEQ ID NO:2 and SEQ ID NO:7; SEQ ID NO:3 and SEQ ID NO:7; SEQ ID NO:4 and SEQ ID NO:7; SEQ ID NO:5 and SEQ ID NO:7; SEQ ID NO:6 and SEQ ID NO:7; SEQ ID NO:17 and SEQ ID NO:7; SEQ ID NO:18 and SEQ ID NO:7; SEQ ID NO:1 and SEQ ID NO:8; SEQ ID NO:2 and SEQ ID NO:8; SEQ ID NO:3 and SEQ ID NO:8; SEQ ID NO:4 and SEQ ID NO:8; SEQ ID NO:5 and SEQ ID NO:8; SEQ ID NO:6 and SEQ ID NO:8; SEQ ID NO:17 and SEQ ID NO:8; SEQ ID NO:18 and SEQ ID NO:8; SEQ ID NO:1 and SEQ ID NO:9; SEQ ID NO:2 and SEQ ID NO:9; SEQ ID NO:3 and SEQ ID NO:9; SEQ ID NO:4 and SEQ ID NO:9; SEQ ID NO:5 and SEQ ID NO:9; SEQ ID NO:6 and SEQ ID NO:9; SEQ ID NO:17 and SEQ ID NO:9; SEQ ID NO:18 and SEQ ID NO:9; SEQ ID NO:1 and SEQ ID NO:10; SEQ ID NO:2 and SEQ ID NO:10; SEQ ID NO:3 and SEQ ID NO:10; SEQ ID NO:4 and SEQ ID NO:10; SEQ ID NO:5 and SEQ ID NO:10; SEQ ID NO:6 and SEQ ID NO:10; SEQ ID NO:17 and SEQ ID NO:10; SEQ ID NO:18 and SEQ ID NO:10; SEQ ID NO:1 and SEQ ID NO:11; SEQ ID NO:2 and SEQ ID NO:11; SEQ ID NO:3 and SEQ ID NO:11; SEQ ID NO:4 and SEQ ID NO:11; SEQ ID NO:5 and SEQ ID NO:11; SEQ ID NO:6 and SEQ ID NO:11; SEQ ID NO:17 and SEQ ID NO:11; SEQ ID NO:18 and SEQ ID NO:11; SEQ ID NO:1 and SEQ ID NO:12; SEQ ID NO:2 and SEQ ID NO:12; SEQ ID NO:3 and SEQ ID NO:12; SEQ ID NO:4 and SEQ ID NO:12; SEQ ID NO:5 and SEQ ID NO:12; SEQ ID NO:6 and SEQ ID NO:12; SEQ ID NO:17 and SEQ ID NO:12; SEQ ID NO:18 and SEQ ID NO:12; SEQ ID NO:1 and SEQ ID NO:13; SEQ ID NO:2 and SEQ ID NO:13; SEQ ID NO:3 and SEQ ID NO:13; SEQ ID NO:4 and SEQ ID NO:13; SEQ ID NO:5 and SEQ ID NO:13; SEQ ID NO:6 and SEQ ID NO:13; SEQ ID NO:17 and SEQ ID NO:13; SEQ ID NO:18 and SEQ ID NO:13; SEQ ID NO:1 and SEQ ID NO:14; SEQ ID NO:2 and SEQ ID NO:14; SEQ ID NO:3 and SEQ ID NO:14; SEQ ID NO:4 and SEQ ID NO:14; SEQ ID NO:5 and SEQ ID NO:14; SEQ ID NO:6 and SEQ ID NO:14; SEQ ID NO:17 and SEQ ID NO:14; SEQ ID NO:18 and SEQ ID NO:14; SEQ ID NO:1 and SEQ ID NO:15; SEQ ID NO:2 and SEQ ID NO:15; SEQ ID NO:3 and SEQ ID NO:15; SEQ ID NO:4 and SEQ ID NO:15; SEQ ID NO:5 and SEQ ID NO:15; SEQ ID NO:6 and SEQ ID NO:15; SEQ ID NO:17 and SEQ ID NO:15; SEQ ID NO:18 and SEQ ID NO:15; SEQ ID NO:1 and SEQ ID NO:16; SEQ ID NO:2 and SEQ ID NO:16; SEQ ID NO:3 and SEQ ID NO:16; SEQ ID NO:4 and SEQ ID NO:16; SEQ ID NO:5 and SEQ ID NO:16; SEQ ID NO:6 and SEQ ID NO:16; SEQ ID NO:17 and SEQ ID NO:16; and SEQ ID NO:18 and SEQ ID NO:16.
33. In some embodiments of the method of paragraphs 3-31, the β-glucanase and the xylanase have an amino acid sequence having at least 80% sequence identity with the respective SEQ ID, or any functional fragment thereof, being selected from the list consisting of SEQ ID NO:1 and SEQ ID NO:7; SEQ ID NO:2 and SEQ ID NO:8; and SEQ ID NO:2 and SEQ ID NO:8.
34. In some embodiments of the method of paragraphs 3-33, the xylanase and the beta-glucanase are combined at a ratio of 1:1 per weight.
35. In some embodiments of the method of paragraphs 3-34, the beta-glucanase has a beta-glucanase activity of at least 9000 U/g, and the xylanase has a xylanase activity of at least 13000U/g.
36. In some embodiments of the method of paragraph 3-31 the β-glucanase and the xylanase comprises commercially available enzymes having xylanase activity and/or beta-glucanase activity selected from the group consisting of UltraFlo L (available from Novozymes -beta glucanase with cellulase, xylanase side activities), UltraFlo XL (available from Novozymes -beta glucanase with xylanase and alpha amylase side activities), UltraFlo Max (available from Novozymes - beta glucanase and xylanase), Finizyme 250 L(available from Novozymes - beta glucanase with cellulase, xylanase side activities) and Filtrase series(available from DSM- betaglucanase and xylanases).
37. In some embodiments of the method according to any preceding paragraph, the further comprises adding one or more enzymes selected from the group of amylase, a protease, naturally occurring enzymes in the cereal and combinations thereof.
38. In some embodiments of the method according to any preceding paragraph, a total steeping time is at least 10 % less than the time of a malted cereal prepared without the β-glucanase and/or the xylanase.
39. In some embodiments of the method according to any preceding paragraph, a total steeping time does not exceed 25 hours.
40. In some embodiments of the method according to any preceding paragraph, a total steeping time does not exceed 12 hours.
41. In some embodiments of the method according to any preceding paragraph, a total amount of water used during the steeping process is at least 10 % less than the water used for a malted cereal prepared without the β-glucanase and/or the xylanase.
42. In some embodiments of the method according to any preceding paragraph, the amount of high molecular weight β-glucans in the malted cereal is at least 10% less than the amount of high molecular weight β-glucans in a malted cereal prepared without the β-glucanase.
43. In some embodiments of the method of paragraphs 3-42, the amount of high molecular weight arabinoxylans is at least 10 % less than the amount of arabinoxylans in a malted cereal prepared without the xylanase.
44. In some embodiments of the method of paragraphs 3-42, the amount of high molecular weight β-glucans and arabinoxylans is at least 50% less than the amount of β-glucans and/or arabinoxylans in a malted cereal prepared without the β-glucanase and/or the xylanase.
45. In some embodiments of the method of paragraphs 2-43, the amount of high molecular weight β-glucans in the malted cereal is 50 mg/l or less, and the amount of arabinoxylans in the malted cereal is at 2000 mg/l or less.
46. In some embodiments of the method of any preceding paragraph, an extract prepared from the malted cereal has lower viscosity that an extract prepared with a malted cereal prepared without the β-glucanase and/or the xylanase.
47. In some embodiments of the method according to any preceding paragraph, the malted cereal has increased enzymatic activity.
48. In some embodiments of the method of paragraph 47, the enzymatic activity is increased by a factor of at least about 2 as compared to a malted cereal prepared without the β-glucanase and/or the xylanase.
49. In some embodiments of the method of paragraphs 47 or 48, the enzymatic activity is selected from the group consisting of β-glucanase activity, xylanase activity, amylase activity, protease activity, naturally occurring enzyme activity in the cereal and combinations thereof.
50. The method of paragraph 47 or 48, wherein the enzymatic activity is β-glucanase and/or xylanase activity.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:

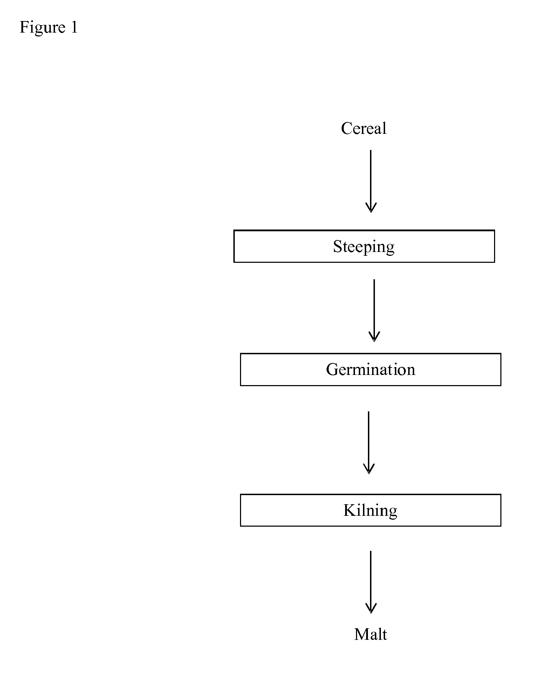

**Figure 1** shows a schematic representation of the malting process. Enzymes described herein can be added one or more times, or constantly, during one or more step of the process.

### DETAILED DESCRIPTION OF THE INVENTION

The present disclosure provides compositions and processes for the preparation of malted cereals, e.g., for the use in production of food and beverages. In some embodiments, the present invention provides methods and compositions for improved preparation of malted cereals, e.g., reduced water consumption and/or germination time. In some embodiments, the present invention provides methods and compositions for improved preparation of malted cereals having higher enzymatic activity. In some embodiments, the present invention provides methods and compositions for improved preparation of malted cereals which improve the processes for food and beverage production, and malted cereals that may improve the properties of said obtained food and beverages.

In one aspect, the present invention is directed to composition and methods for the preparation of malted cereals comprising adding one or more enzymes to the malting process. In some embodiments, the present invention provides compositions and methods for the preparation of malted cereals comprising adding a cell wall degrading enzyme alone or in combination with other enzymes at some point during the malting process. In some embodiments, the cell wall degrading enzyme is a xylanase or a beta glucanase. In one embodiment, the present invention provides compositions and methods for the preparation of malted cereals comprising adding a beta glucanase alone or in combination with other enzymes at some point during the malting process. In one embodiment, the present invention provides compositions and methods for the preparation of malted cereals comprising adding a xylanase alone or in combination with other enzymes at some point during the malting process. In some embodiments, the other enzymes are other cell wall degrading enzymes. In some embodiments the invention provides compositions and methods for the preparation of malted cereals comprising adding a beta glucanase and a xylanase alone or in combination with other enzymes during the malting process. Examples of cereals that can be used in the methods described herein include but are not limited to barley, wheat, rye, corn, oats, rice, millet triticale, cassava, sorghum and the like.

Cereals, such as barley, wheat, rye, corn, oats, rice, millet triticale, cassava, and sorghum, used in the production of food and beverages (e.g. beer) contain varying levels of β-glucans and arabinoxylans. (1,3;1,4)-β-D-Glucans consist of unbranched and unsubstituted chains of (1,3)- and (1,4)-β-glucosyl residues. The (1,3;1,4)-β-D-glucans are most abundant in cell walls of the cereals, specifically in the starchy endosperm of grain, where they can contribute up to 70% by weight of the cell walls in barley, rye, and oats. Arabinoxylan is a hemicellulose found in both the primary and secondary cell walls of cereal grains, consisting of copolymers of two pentose sugars - arabinose and xylose. The arabinose moiety may be further substituted.

Studies have shown that the content of β-glucans and arabinoxylans in the cell wall might be related with grain hardness as well as water uptake of the cereals (e.g. barley). Kernel hardness is one of the major factors affecting the processing and product quality of the grain. A high β-glucans and arabinoxylans content in the cereal may lead to insufficient degradation of cell walls, which in turns hinders the diffusion of germination enzymes and the mobilization of kernel reserves, and hence reduces the malt extract. Further studies have suggested that that residual β-glucans may lead to highly viscous wort, giving rise to filtration problems, and it may participate in causing chill haze.

Without intending to be limited to any theory, in some embodiments, the present invention provides compositions and methods to break down betaglucans and other cell wall components such as arabinoxylans in the grains during the malting process. By breaking down the betaglucans and other cell wall components (e.g., arabinoxylans) in the grain during the malting process the present invention allows for usage of less water during the malting process by decreasing the water binding capacity of beta-glucans and arabinoxylans in the cell wall of the grain while allowing a better and faster water uptake by the grain. Further by breaking down the beta-glucans and other cell wall components (e.g., arabinoxylans) in the grain, the present invention allows for better diffusion of one or more enzymes in the cereal and for better substrate accessibility for said enzymes. Thus, in some embodiments, the present invention provides composition and methods to decrease the amount of water needed during malting (e.g. for steeping). In some embodiments, the present invention provides composition and methods to improve the water uptake by the grain. Further, in some embodiments, the present invention provides composition and methods to decrease the time needed for steeping and the time needed to cause the grains to germinate. In some embodiments, the present invention provides methods and compositions for improved preparation of malted cereals having higher enzymatic activity.

In some embodiments, the preparation process of malted cereals comprises the following steps: a steeping step including one or more wetting stages, a germination step, a drying step (e.g. kilning) including one or more temperature, and one or more enzymes as described herein which are added one or more times during the process. In some embodiments, the same or different one or more enzymes are added in one or more of the wetting stages of the steeping process.

The invention further provides composition and methods for improved preparation of malted cereals having higher enzymatic activity (e.g. betaglucanase and xylanase activity), which can then improve downstream processes for food and beverage production (e.g. easier and more effective processing of the malt in wort and beer production). Thus, in some embodiments, the invention provides enzymes suitable for the production of food and beverage products, such as in the production of an alcoholic or non-alcoholic beverage, such malt-based beverage like beer or whiskey, malted shakes, malt vinegar, flavored drinks such as Malta, Horlicks, Ovaltine and Milo, and in the production of confections such as Maltesers and Whoppers, and some baked goods, such as malt loaf, bagels and rich tea biscuits. The improved properties of the malted cereals comprise e.g. improved temperature optimums, improved ratio in activity on soluble (WE-AX) to insoluble (WU-AX) arabinoxylan substrates, reduced total pressure built up during mash separation and/or filtration steps of a brewing process, as well as increased filterability of enzyme treated material.

As used herein, the terms " beverage" and " beverage(s) product" includes such foam forming fermented beverages as beer brewed with 100% malt, beer brewed under different types of regulations, ale, dry beer, near beer, light beer, low alcohol beer, low calorie beer, porter, bock beer, stout, malt liquor, non-alcoholic beer, non-alcoholic malt liquor and the like. The term "beverages" or "beverages product" also includes non-foaming beer and alternative malt beverages such as fruit flavored malt beverages, e. g. , citrus flavored, such as lemon-, orange-, lime-, or berry-flavored malt beverages, liquor flavored malt beverages, e. g. , vodka-, rum-, or tequila-flavored malt liquor, or coffee flavored malt beverages, such as caffeine-flavored malt liquor, and the like.

Beer is traditionally referred to as an alcoholic beverage derived from malt, such as malt derived from barley grain, and optionally adjunct, such as starch containing plant material (e.g. cereal grains) and optionally flavored, e.g. with hops. In the context of the present invention, the term "beer" includes any fermented wort, produced by fermentation/brewing of a starch-containing plant material, thus in particular beer produced from malt and beer produced from any combination of malt and adjunct. Beer can be made from a variety of starch-containing plant material by essentially the same process, where the starch consists mainly of glucose homopolymers in which the glucose residues are linked by alpha-1, 4- or alpha-1,6-bonds, with the former predominating.

The term "germination" or "germinate" or any other equivalent as used herein means the beginning or resumption of growth by a seed. In accordance with the processes described herein, germination begins to occur during and/or after the cereal has been steeped. Germination of cereals is generally understood to mean hydration of the seed, swelling of the cereal and inducing growth of the embryo. Environmental factors affecting germination include moisture, temperature and oxygen level. A rapid increase in cells of the root stem leads to root development, while corresponding growth sends forth a shoot.

As used herein, the term "steeping" refers to wetting of the cereal. Wetting may include one or more stages over a time and temperature effective for providing a moisture content desired for a malting process, e.g., between about 20% and about 60% by weight, or between 40% to about 50%. Wetting may also including constant spraying of the cereal, or spraying at one or more stages during the malting process (e.g., during the germination step).

Unless defined otherwise, all technical and scientific terms used herein have the same meaning as is commonly understood by one of skill in the art to which this invention belongs. All patents and publications referred to herein are incorporated by reference.

### Enzymes

In one aspect, the present invention is directed to composition and methods for the preparation of malted cereals comprising adding one or more enzymes to the malting process. In one embodiment, the present invention provides compositions and methods for the preparation of malted cereals comprising adding a beta glucanase and/or a xylanase alone or in combination with other enzymes during the malting process.

In one embodiment, the present invention provides compositions and methods for the preparation of malted cereals comprising adding a beta glucanase alone or in combination with other enzymes during the malting process.

In some embodiments, the enzyme for the methods and compositions described herein may have cellulolytic activity. The systematic name of cellulose is (1,3;1,4)-beta-D-glucan. 4-glucanohydrolase and cellulolytic enzymes or cellulases are classified in EC 3.2.1.4. Cellulases endohydrolyse (1 4)- beta- D-glucosidic linkages (in, e.g., cellulose, and lichenin), beta--D-glucans, and will also hydrolyse 1,4-linkages in β-D-glucans (also containing 1,3-linkages). Cellulase also have other names such as endo-1,4-beta-D-glucanase, beta-1,4-glucanase, beta-1,4-endoglucan hydrolase, cellulase A, cellulosin AP, endoglucanase D, alkali cellulose, cellulase A 3, celludextrinase, 9.5 cellulase, avicelase, pancellase SS and 1,4-(1,3;1,4)-beta-D-glucan 4-glucanohydrolase.

In one aspect of the invention, the cellulase activity of the enzyme composition according to the invention can be measured by the cellulase activity assays as described herein or by any feasible method known in the art.

In further aspects, the present invention relates to enzymes having endo-1,3(4)-beta-glucanase activity, which can be determined by the assays described herein or by any feasible method known in the art.

"β-glucanase" or "beta-glucanase" as used herein refers to an endo-1,3(4)-beta-glucanase of EC 3.2.1.6. β-glucanases catalyze the endohydrolysis of (1→3)- or (1→4)-linkages in beta-D-glucans. Suitable beta-glucanases to be used alone or in combination with one or more cell wall degrading enzyme (e.g., an enzyme exhibiting endo-1,4-beta-xylanase activity) according to the invention includes any one betaglucanase disclosed in WO2004087889, WO2005059084, WO9414953, WO2007056321, WO9531533, WO08023060, WO2005100582, WO9828410, WO9742301, WO2006066582, WO05118769, WO2005003319, and WO10059424.

Typically, standard assays are carried out at pH 5.0, however, the assays can be performed at different pH values for the additional characterization and specification of enzymes.

One unit of endo-1,3(4)-beta-glucanase activity is defined as the amount of enzyme which produces 1 µmole glucose equivalents per minute under the conditions of the assay (e.g., pH 5.0 (or as specified) and 50 °C).

In some embodiments, the enzyme for the methods and compositions described herein comprises β-glucanase activity of at least about 10000 U/g, such as at least about 12000 U/g, such as at least about 14000 U/g, such as at least about 15000 U/g, such as at least about 18000 U/g as measured by the assays described herein or by any feasible method known in the art. In some embodiments, a different β-glucanase activity is used depending of the β-glucan content in the cereal and/or the malting conditions and/or the malted cereal requirements.

In further aspects, the enzyme for the methods and compositions described herein has laminarinase activity or comprises any one or more further enzyme having laminarinase activity. The laminarinase activity can be determined as described in the laminarase assays herein or by any feasible method known in the art.

Laminarinase may be an endo-1,3(4)-beta-glucanase classified in E.C. 3.2.1.6 or glucan endo-1,3-beta-D-glucosidase classified in E.C. 3.2.1.39. Endo-1,3(4)-beta-glucanase with the alternative names, laminarinase, endo-1,3-beta-glucanase. Endo-1,4-beta-glucanase is classified in E.C. 3.2.1.6. The substrates include laminarin, lichenin and cereal D-glucans. The enzyme catalyzes endohydrolysis of (1→3)- or (1→4)-linkages in beta-D-glucans when the glucose residue whose reducing group is involved in the linkage to be hydrolyzed is itself substituted at C-3. Glucan endo-1,3-beta-D-glucosidase with the alternative names (1→3)-beta-glucan endohydrolase, Endo-1,3-beta-glucanase and laminarinase is classified in E.C. 3.2.1.39. Glucan endo-1,3-beta-D-glucosidase hydrolyses (1→3)-beta-D-glucosidic linkages in (1→3)-beta-D-glucans in substrates as e.g. laminarin, paramylon and pachyman.

In some aspects, the enzyme for the methods and compositions described herein has xyloglucan-specific exo-beta-1,4-glucanase activity or comprises a further enzyme having xyloglucan-specific exo-beta-1,4-glucanase activity, "xyloglucan-specific exo-beta-1,4-glucanase" refers to enzymes of E.C3.2.1.155. Xyloglucan-specific exo-beta-1,4-glucanase catalyze the exohydrolysis of (1→4)-beta-D-glucosidic linkages in xyloglucan.

In one embodiment, the present invention provides compositions and methods for the preparation of malted cereals comprising adding a beta glucanase alone or in combination with other enzymes during the malting process. In some embodiments, the other enzymes are cell wall degrading enzymes. In some embodiments, the cell wall degrading enzyme is a xylanase. Thus, in some embodiments the invention provides compositions and methods for the preparation of malted cereals comprising adding a beta glucanase and a xylanase alone or in combination with other enzymes during the malting process.

In one embodiment, the present invention provides compositions and methods for the preparation of malted cereals comprising adding a xylanase alone or in combination with other enzymes during the malting process. In some embodiments, the other enzymes are cell wall degrading enzymes.

Xylanases are classified in EC 3.2.1.8, EC 3.2.1.32, EC 3.2.1.136 and EC 3.2.1.156.; their activity may be measured e.g. as described herein or by any suitable method known in the art. Suitable xylanases to be used in combination with an enzyme exhibiting endo-1,3(4)-beta-glucanase activity according to the invention includes any xylanase classified in EC 3.2.1.8, EC 3.2.1.32, EC 3.2.1.136 and EC 3.2.1.156, such as any of the ones disclosed in WO 2010072226, WO 2010072225, WO2010072224, WO 2005059084, WO2007056321, WO2008023060A, WO9421785, WO2006114095, WO2006066582, US 2008233175, and WO10059424.

Endo-1,4-beta xylanase is classified as EC 3.2.1.8. The enzyme causes endohydrolysis of 1,4-beta-D-xylosidic linkages in xylans.

The terms "family 11 xylanase", "Glycoside hydrolase (GH) family 11" or simply "GH 11 xylanase" as used herein refers to an endo-1,4-beta xylanase classified as EC 3.2.1.8, which causes endohydrolysis of 1,4-beta-D-xylosidic linkages in xylans and which is classified as a family 11 xylanase according to B. Henrissat , A classification of glycosyl hydrolases based on amino acid sequence similarities. Biochem. J. 280 (1991), pp. 309-316.

The terms "Family 10 xylanase", "Glycoside hydrolase (GH) family 10", or simply "GH 10 xylanase" comprises enzymes with a number of known activities, such as xylanase (EC:3.2.1.8); endo-1,3-beta-xylanase (EC:3.2.1.32); cellobiohydrolase (EC:3.2.1.91). These enzymes were formerly known as cellulase family F.

In some embodiments the enzyme exhibiting endo-1,4-beta-xylanase activity is a family 11 xylanase. In some embodiments the enzyme exhibiting endo-1,4-beta-xylanase activity is a family 10 xylanase.

In some embodiments, the enzyme exhibiting xylanase activity is a family 5 xylanase. The term "Family 5 xylanase" (formerly family A) includes glycoside hydrolases with varying activities, including: endoglycosylceramidase (EC 3.2.1.123), cellulase (EC 3.2.1.4), licheninase (EC3.2.1.73), beta-mannosidase (EC 3.2.1.25), glucan 1,3-bglucosidase (EC 3.2.1.58), glucan endo-1,6-beta-glucosidase (EC 3.2.1.75), mannan endo-1,4-beta-mannosidase (EC 3.2.1.58), cellulose 1,4-beta-cellobiosidase (EC3.2.1.91), endo-1,6-beta-galactanase (EC 3.2.1.-), 1,3-mannanase (EC 3.2.1.-) and endo-1,4-beta-xylanase (EC3.2.1.8).

In one aspect, the enzyme composition according to the invention has endo-1,4-beta xylanase activity as measured by the assays described herein or any suitable assay known in the art.

An assay for measuring xylanase activity may be carried out at pH 3.5 or pH 5 and 50 °C using xylan as substrate, or it can be performed at different pH and temperature values for the additional characterization and specification of enzymes. Enzyme activity can be calculated from the increase in absorbance caused by xylose at 540 nm per unit time.

In some embodiments the enzyme composition according to the invention comprises a xylanase activity of at least about 5000 U/g, such as at least about 6000 U/g, such as at least about 7000 U/g, such as at least about 8000 U/g, such as at least about 8500 U/g, as measured by in the assays described herein or any suitable assay known in the art. In some embodiments, a different β-xylanase activity is used, e.g., depending of the arabinoxylan content in the cereal and/or the malting conditions and/or the malted cereal requirements.

In some aspects, the enzyme composition according to the invention has arabinanase activity or comprises a further enzyme having arabinanase activity. Arabinanase is classified as EC3.2.1.99. The systematic name is 5-alpha-L-arabinan 5-alpha-L-arabinanohydrolase but it has several other names such as arabinan endo-1,5-alpha-L-arabinosidase, and endo-1,5-alpha-L-arabinanase, endo-alpha-1,5-arabanase, endo-arabanase, 1,5-alpha-L-arabinan and 1,5-alpha-L-arabinanohydrolase. Arabinase endohydrolyses (1→5)-alpha-arabinofuranosidic linkages in (1→5)-arabinans. Arabinanase also acts on arabinan.

In one aspect of the invention, the arabinase activity of the enzyme composition according to the invention is measured by arabinase assay as herein or any suitable method known in the art. The assay can be carried out at pH 3.5 and 50 °C using sugar beet arabinan as substrate, and it can be performed at different pH and temperature values for the additional characterization and specification of enzymes. Enzyme activity can be calculated from the increase in absorbance at 540 nm per unit time.

One unit of arabinase activity is defined as the amount of enzyme (normalized for total assay volume) that gives an increase in absorbance under the conditions of the assay used (e.g., pH 3.5 and 50 °C).

In some aspects, the enzyme composition according to the invention has beta-D-glucoside glucohydrolase activity or comprises a further enzyme having beta-D-glucoside glucohydrolase activity. Beta-D-glucoside glucohydrolase refers to enzymes of E.C 3.2.1.21.

In some aspects, the enzyme composition according to the invention has β-Xylosidase activity or comprises a further enzyme having β-Xylosidase activity. " β-Xylosidase" or "Xylan 1,4-beta-xylosidase" refers to enzymes of E.C 3.2.1.37. β-Xylosidase catalyze the hydrolysis of (1→4)-beta-D-xylans, to remove successive D-xylose residues from the non-reducing termini.

In some aspects of the invention, the enzyme composition according to the invention has cellobiohydrolase activity or comprises a further enzyme having cellobiohydrolase activity. "Cellobiohydrolase" or "Cellulose 1,4-beta-cellobiosidase" refers to enzymes of EC 3.2.1.91. Cellulose 1,4-beta-cellobiosidase catalyzes hydrolysis of 1,4-beta-D-glucosidic linkages in cellulose and cellotetraose, releasing cellobiose from the non-reducing ends of the chains.

The cellobiohydrolase activity of the enzyme composition according to the invention is measured by the cellobiohydrolase assays as described herein or any suitable assay known in the art. The standard assay is carried out at pH 5.0, and it can be performed at different pH values for the additional characterization and specification of enzymes.

One unit of cellobiohydrolase activity is defined as the amount of enzyme which produces 1 µmole p-nitrophenol from p-nitrophenyl b-D-cellobiopyranoside per minute under the conditions of the assay (e.g., pH 5.0 (or as specified) and 50 °C).

In some aspects, the enzyme composition according to the invention has alpha-N-arabinofuranosidase activity or comprises a further enzyme having arabinofuranosidase activity. "alpha-N-arabinofuranosidase" or "Alpha-N-arabinofuranosidase" refers to enzymes of EC 3.2.1.55. alpha-N-arabinofuranosidase catalyzes the hydrolysis of terminal non-reducing alpha-L-arabinofuranoside residues in alpha-L- arabinosides.

In one aspect of the invention, the arabinofuranosidase activity of the enzyme composition according to the invention is measured by the arabinofuranosidase assay as described herein or by any suitable assay known in the art. The standard assay can be carried out at pH 5.0 and 50 °C and it can be performed at different values of pH and temperatures for the additional characterization and specification of enzymes.

One unit of alpha-N-arabinofuranosidase activity is defined as the amount of enzyme which produces 1 µmole p-nitrophenol from p-nitrophenyl alpha-L-arabinofuranoside per minute under the conditions of the assay (e.g., pH 5.0 and 50 °C (or as specified)).

In some aspects, the enzyme composition according to the invention has glucan 1,4-betaglucosidase activity or comprises a further enzyme having glucan 1,4-beta-glucosidase activity. "Glucan 1,4-beta-glucosidase" or "glucan 1,4-beta-glucosidase" refers to enzymes of E.C3.2.1.74. Glucan 1,4-beta-glucosidase catalyze the hydrolysis of (1→4)-linkages in (1→4)-beta-D-glucans, to remove successive glucose units.

In some embodiments, one or more enzyme exhibiting endo-1,4-beta-xylanase activity and/or one or more enzyme exhibiting beta-glucanase activity, alone or in combination, provide for improved malting processes.

In some embodiments, one or more enzyme exhibiting endo-1,4-beta-xylanase activity and/or one or more enzyme exhibiting beta-glucanase activity, alone or in combination, provide for a decrease in water consumption during the steeping process.

In some embodiments, one or more enzyme exhibiting endo-1,4-beta-xylanase activity and/or one or more enzyme exhibiting beta-glucanase activity, alone or in combination, provide for improved and/or faster intake of water by the grain during the steeping process.

In some embodiments, one or more enzyme exhibiting endo-1,4-beta-xylanase activity and/or one or more enzyme exhibiting beta-glucanase activity, alone or in combination, provide a decrease in the time needed for steeping.

In some embodiments, one or more enzyme exhibiting endo-1,4-beta-xylanase activity and/or one or more enzyme exhibiting beta-glucanase activity, alone or in combination, provide for a decrease in the time needed to cause the grains to germinate.

In some embodiments, one or more enzyme exhibiting endo-1,4-beta-xylanase activity and/or one or more enzyme exhibiting beta-glucanase activity, alone or in combination, provide for a malted cereal with increase enzymatic activity (e.g., xylanase activity and/or beta-glucanase activity).

In some embodiments, one or more enzyme exhibiting endo-1,4-beta-xylanase activity and/or one or more enzyme exhibiting beta-glucanase activity, alone or in combination, provide for better diffusion of one or more cereal enzymes and for better substrate accessibility for said enzymes.

In some embodiments, one or more enzyme exhibiting endo-1,4-beta-xylanase activity and/or one or more enzyme exhibiting beta-glucanase activity, alone or in combination, provide for a malted cereal with a decrease amount of high molecular weight β-glucans and arabinoxylans.

In some embodiments, one or more enzyme exhibiting endo-1,4-beta-xylanase activity and/or one or more enzyme exhibiting beta-glucanase activity, alone or in combination, provide for a malted cereal with lower viscosity.

In some embodiments, one or more enzyme exhibiting endo-1,4-beta-xylanase activity and/or one or more enzyme exhibiting beta-glucanase activity, alone or in combination, provide for a malted cereal with a lower potential for off flavor formation, such as off flavor formation related to arabinoxylan breakdown.

In some embodiments, one or more enzyme exhibiting endo-1,4-beta-xylanase activity and/or one or more enzyme exhibiting beta-glucanase, alone or in combination, provide for a malted cereal with better filterability, such as mash separation and beer filtration.

One aspect of the invention relates to an enzyme exhibiting endo-1,4-beta-xylanase activity, which enzyme comprises an amino acid sequence having at least 80% identity with any one selected from SEQ ID NO:1, SEQ ID NO:2, SEQ ID NO:3, SEQ ID NO:4, SEQ ID NO:5, SEQ ID NO:6, SEQ ID NO:17, and SEQ ID NO:18, or any functional fragment thereof.

As used herein "functional fragment" refers to a truncated version of an enzyme with essentially the same or at least a significant degree of enzyme activity as the non-truncated reference enzyme.

Another aspect relates to an enzyme exhibiting endo-1,3(4)-beta-glucanase activity, which enzyme comprises an amino acid sequence having at least 80% identity with any one selected from SEQ ID NO:7, SEQ ID NO:8, SEQ ID NO:9, SEQ ID NO:10, and SEQ ID NO:11, SEQ ID NO:12, SEQ ID NO:13, SEQ ID NO:14, SEQ ID NO:15, SEQ ID NO:16, or any functional fragment thereof.

In some embodiments, the enzyme has a temperature optimum in the range of 5-80 °C, such as in the range of 5-40°C or 15-80°C, such as in the range 20-80 °C, such as in the range 5-15°C, 15-20°C, 45-65 °C, 50-65 °C, 55-65 °C or 60-80°C.

In some embodiments, the enzyme has at least 81, 82, 83, 84, 85, 86, 87, 88, 89, 90, 91, 92, 93, 94, 95, 96, 97, 98 or 99% identity with any one amino acid sequence selected from SEQ ID NO: 1-18, or any functional fragment thereof.

In some embodiments, the enzyme has a total number of amino acids of less than 350, such as less than 340, such as less than 330, such as less than 320, such as less than 310, such as less than 300 amino acids, such as in the range of 200 to 350, such as in the range of 220 to 345 amino acids.

In some embodiments, the amino acid sequence of the enzyme has at least one, two, three, four, five, six, seven, eight, nine or ten amino acid substitutions as compared to any one amino acid sequence selected from SEQ ID NO: 1-18, or any functional fragment thereof.

In some embodiments, the amino acid sequence of the enzyme has a maximum of one, two, three, four, five, six, seven, eight, nine or ten amino acid substitutions compared to any one amino acid sequence selected from SEQ ID NO: 1-18, or any functional fragment thereof.

In some embodiments, the enzyme comprises the amino acid sequence identified by any one of SEQ ID NO: 1-18, or any functional fragment thereof.

In some embodiments, the enzyme consists of the amino acid sequence identified by any one of SEQ ID NO: 1-18, or any functional fragment thereof.

A further important aspect of the invention relates to compositions and methods comprising an enzyme exhibiting endo-1,4-p -xylanase activity in combination with any one or more beta-glucanase as described herein.

A further important aspect of the invention compositions and methods comprising an enzyme exhibiting endo-1,3(4)-beta-glucanase activity in combination with any one or more xylanase as described herein. In some embodiments this one or more xylanase is an enzyme according to SEQ ID NO: 17 and/or SEQ ID NO: 18, or any functional fragment thereof.

In some embodiments the combination of an enzyme exhibiting endo-1,4-beta-xylanase activity with an enzyme exhibiting endo-1,3(4)-beta-glucanase activity is according to the following table:

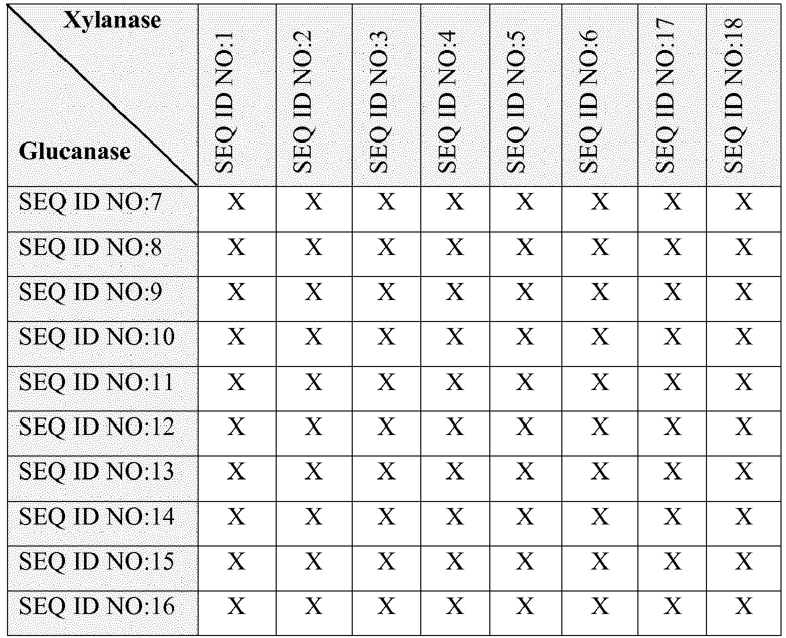

It is to be understood that any one of the above combination of an enzyme being an enzyme exhibiting endo-1,4-beta-xylanase activity with an enzyme exhibiting endo-1,3(4)-beta-glucanase activity may be combined with a ratio between the two enzymes of 1:10, 2:10, 3:10, 4:10, 5:10, 6:10, 7:10, 8:10, 9:10, 10:10, 10:9, 10:8, 10:7, 10:6, 10:5, 10:4, 10:3, 10:2, or 10:1, such as within a range of 1:10-10:1, such as 2:10-10:2, such as 3:10-10:3, such as 4:10-10:4, such as 5:10-10:5, such as 6:10-10:6, such as 7:10-10:7, such as 8:10-10:8, or within 9:10-10:9. It is to be understood that any one of the above combination of an enzyme being an enzyme exhibiting endo-1,4-beta-xylanase activity with an enzyme exhibiting endo-1,3(4)-beta-glucanase activity may be combined with a ratio between the two enzymes of 1:1.

In some embodiments the composition according to the invention comprises a combination of at least two enzymes, said two enzymes, or two enzymes with an amino acid sequence having at least 80% sequence identity with the respective SEQ ID, or any functional fragment thereof, being selected from the list consisting of: SEQ ID NO 1 and SEQ ID NO:7; SEQ ID NO 2 and SEQ ID NO:7; SEQ ID NO 3 and SEQ ID NO:7; SEQ ID NO 4 and SEQ ID NO:7; SEQ ID NO 5 and SEQ ID NO:7; SEQ ID NO 6 and SEQ ID NO:7; SEQ ID NO 17 and SEQ ID NO:7; SEQ ID NO 18 and SEQ ID NO:7; SEQ ID NO 1 and SEQ ID NO:8; SEQ ID NO 2 and SEQ ID NO:8; SEQ ID NO 3 and SEQ ID NO:8; SEQ ID NO 4 and SEQ ID NO:8; SEQ ID NO 5 and SEQ ID NO:8; SEQ ID NO 6 and SEQ ID NO:8; SEQ ID NO 17 and SEQ ID NO:8; SEQ ID NO 18 and SEQ ID NO:8; SEQ ID NO 1 and SEQ ID NO:9; SEQ ID NO 2 and SEQ ID NO:9; SEQ ID NO 3 and SEQ ID NO:9; SEQ ID NO 4 and SEQ ID NO:9; SEQ ID NO 5 and SEQ ID NO:9; SEQ ID NO 6 and SEQ ID NO:9; SEQ ID NO 17 and SEQ ID NO:9; SEQ ID NO 18 and SEQ ID NO:9; SEQ ID NO 1 and SEQ ID NO:10; SEQ ID NO 2 and SEQ ID NO:10; SEQ ID NO 3 and SEQ ID NO:10; SEQ ID NO 4 and SEQ ID NO:10; SEQ ID NO 5 and SEQ ID NO:10; SEQ ID NO 6 and SEQ ID NO:10; SEQ ID NO 17 and SEQ ID NO:10; SEQ ID NO 18 and SEQ ID NO:10; SEQ ID NO 1 and SEQ ID NO:11; SEQ ID NO 2 and SEQ ID NO:11; SEQ ID NO:3 and SEQ ID NO:11; SEQ ID NO:4 and SEQ ID N0:11; SEQ ID NO:5 and SEQ ID N0:11; SEQ ID NO:6 and SEQ ID N0:11; SEQ ID NO:17 and SEQ ID N0:11; SEQ ID NO:18 and SEQ ID NO:11; SEQ ID NO:1 and SEQ ID NO:12; SEQ ID NO:2 and SEQ ID NO:12; SEQ ID NO:3 and SEQ ID NO:12; SEQ ID NO:4 and SEQ ID NO:12; SEQ ID NO:5 and SEQ ID NO:12; SEQ ID NO:6 and SEQ ID NO:12; SEQ ID NO:17 and SEQ ID NO:12; SEQ ID NO:18 and SEQ ID NO:12; SEQ ID NO:1 and SEQ ID NO:13; SEQ ID NO:2 and SEQ ID NO:13; SEQ ID NO:3 and SEQ ID NO:13; SEQ ID NO:4 and SEQ ID NO:13; SEQ ID NO: and SEQ ID NO: 13; SEQ ID NO:6 and SEQ ID NO:13; SEQ ID NO:17 and SEQ ID NO:13; SEQ ID NO:18 and SEQ ID NO:13; SEQ ID NO:1 and SEQ ID NO:14; SEQ ID NO:2 and SEQ ID NO:14; SEQ ID NO:3 and SEQ ID NO:14; SEQ ID NO:4 and SEQ ID NO:14; SEQ ID NO:5 and SEQ ID NO:14; SEQ ID NO:6 and SEQ ID NO:14; SEQ ID NO:17 and SEQ ID NO:14; SEQ ID NO:18 and SEQ ID NO:14; SEQ ID NO:1 and SEQ ID NO:15; SEQ ID NO:2 and SEQ ID NO:15; SEQ ID NO:3 and SEQ ID NO:15; SEQ ID NO:4 and SEQ ID NO:15; SEQ ID NO:5 and SEQ ID NO:15; SEQ ID NO:6 and SEQ ID NO:15; SEQ ID NO:17 and SEQ ID NO:15; SEQ ID NO:18 and SEQ ID NO:15; SEQ ID NO:1 and SEQ ID NO:16; SEQ ID NO:2 and SEQ ID NO:16; SEQ ID NO:3 and SEQ ID NO:16; SEQ ID NO:4 and SEQ ID NO:16; SEQ ID NO:5 and SEQ ID NO:16; SEQ ID NO:6 and SEQ ID NO:16; SEQ ID NO:17 and SEQ ID NO:16; and SEQ ID NO:18 and SEQ ID NO:16.

In some embodiments, the methods comprise an enzyme having xylanase activity of SEQ ID NO:1 and an enzyme exhibiting endo-1,3(4)-β-glucanase activity of SEQ ID NO:7. In some embodiments, the methods comprise an enzyme having xylanase activity of SEQ ID NO:2 and an enzyme exhibiting endo-1,3(4)-β-glucanase activity of SEQ ID NO:8. In some embodiments, the methods comprise an enzyme having xylanase activity of SEQ ID NO:2 and an enzyme exhibiting endo-1,3(4)-β-glucanase activity of SEQ ID NO:8. In some embodiments, any of the above combination of an enzyme being an enzyme exhibiting endo-1,4-beta-xylanase activity with an enzyme exhibiting endo-1,3(4)-beta-glucanase activity may be combined with a ratio between the two enzymes of 1:1 per weight. In some embodiments, any of the above combination, comprise an enzyme exhibiting endo-1,3(4)-beta-glucanase activity having a minimum activity of 9000 U/g and an enzyme exhibiting endo-1,4-beta-xylanase activity having a minimum activity of 13000U/g.

In some embodiments the composition and methods according to the invention comprises the use of any suitable commercially available enzymes having xylanase activity and/or beta-glucanase activity such as UltraFlo L (available from Novozymes -beta glucanase with cellulase, xylanase side activities), UltraFlo XL (available from Novozymes -beta glucanase with xylanase and alpha amylase side activities), UltraFlo Max (available from Novozymes - beta glucanase and xylanase), Finizyme 250 L(available from Novozymes - beta glucanase with cellulase, xylanase side activities) Filtrase series(available from DSM- beta-glucanase and xylanases).

In some embodiments the endo-1,3(4)-beta-glucanase activity and the endo-1,4-beta-xylanase activity are derived from at least two different enzymes. In some embodiments, the two different enzymes are from two different species.

In some embodiments the composition according to the invention comprises any one or more further enzyme. In some embodiments the one or more further enzyme is selected from a list consisting of a xylanase classified in EC 3.2.1.32, EC 3.2.1.136, or EC 3.2.1.156, a cellulase, a laminarinase, an endo-1,5-a-L-arabinanase, a beta-D-glucoside glucohydrolase, a beta -Xylosidase, a cellobiohydrolase, a glucan 1,4-beta-glucosidase, a xyloglucan-specific exobeta-1,4-glucanase and an alpha Arabinofuranosidase.

Sequences and enzymes identified by a sequence as mentioned herein and used according to the present invention alone or in combinations with other enzymes or compounds may be with or without signal peptide.

### Methods

In one aspect, the present invention is directed to methods for the preparation of malted cereals comprising adding one or more enzymes to the malting process. In some embodiments, the present invention provides methods for the preparation of malted cereals comprising adding a cell wall degrading enzyme alone or in combination with other enzymes at some point during the malting process. In some embodiments, the cell wall degrading enzyme is a xylanase or a beta glucanase. In one embodiment, the present invention provides methods for the preparation of malted cereals comprising adding a beta glucanase alone or in combination with other enzymes at some point during the malting process. In one embodiment, the present invention provides compositions and methods for the preparation of malted cereals comprising adding a xylanase alone or in combination with other enzymes at some point during the malting process. In some embodiments, the other enzymes are other cell wall degrading enzymes. In some embodiments the invention provides compositions and methods for the preparation of malted cereals comprising adding a beta glucanase and a xylanase alone or in combination with other enzymes during the malting process.

In some embodiments, the present invention provides methods for the preparation process of malted cereals comprising the following steps: a steeping step including one or more wetting stages, a germination step, a drying step (e.g. kilning) including one or more temperature, and one or more enzymes as described herein which are added one or more times during the process. In some embodiments, the same or different one or more enzymes are added in one or more of the wetting stages of the steeping process. In some embodiments, the steeping step and/or the germination step involve spraying the cereal, and one or more enzymes as described herein are added one or more times during the process. In some embodiments, the one or more enzymes as described herein are added constantly during the spraying of the cereal.

In some embodiments, the cereals to be malted according to the methods described herein are selected from the group consisting of barley, wheat, rye, corn, oats, rice, millet, triticale, cassava, sorghum and a combination thereof.

The one or more enzymes described herein (e.g. a beta glucanase and/or a xylanase) may be introduced before or during the malting process. For example, the one or more enzymes described herein (e.g. a beta glucanase and/or a xylanase) may be introduced during the various malting stages before or after steeping of the cereal. In some embodiments the one or more enzymes described herein are added at the final stages of the malting process or after the malting process is completed to further increase the enzymatic activity of the malt.

In some embodiments, the preparation process of malted cereals comprises a steeping step including one or more wetting stages, where one or more enzymes as described herein are added one or more times during one or more of wetting stages. In some embodiments, the same or different enzymes are added one or more times during one or more of wetting stages of the steeping process. In some embodiments, the one or more wetting stages involve spraying the cereal, where one or more enzymes as described herein are added one or more times or constantly during the spraying.

The concentration of one or more enzymes described herein (e.g., a beta glucanase and/or a xylanase) may vary depending on the conditions of the malting process, the type of cereal (e.g., β-glucans and arabinoxylans content in the grain), the desired specification of the final malted product (e.g. viscosity, β-glucans and arabinoxylans content and final enzyme activity in the malt product, etc....) and the type of enzyme(s) being utilized. Generally about 0.01-100 mg enzyme protein per kilogram of cereals per gram air dry cereal is utilized. In some embodiments, about 0.1-50 mg/kg, or about 1-15 mg/kg or about 10-100 mg/kg are utilized. In some embodiments, about 3.75 mg/kg, or about 5 mg/kg, or about 6.25 mg/kg; or about 7.5 mg/kg; or about 10 mg/kg or about 12.5 mg/kg are utilized. In some embodiments, about 10 U/kg - 1000 U/kg are utilized. In some embodiments, about 10 U/kg - 100 U/kg, or about 30 U/kg - 100 U/kg, or about 50 U/kg - 100 U/kg are utilized. In some embodiments about 100 U/kg - 1000 U/kg are utilized.

In some embodiments, the methods comprise an enzyme having xylanase activity selected from the group consisting of SEQ ID NO:1, SEQ ID NO:2, SEQ ID NO:3, SEQ ID NO:4, SEQ ID NO:5, SEQ ID NO:6, SEQ ID NO:17, and SEQ ID NO:18 , or any functional fragment thereof. In some embodiments, the methods comprise an enzyme exhibiting endo-1,3(4)-β-glucanase activity selected from the group consisting of SEQ ID NO:7, SEQ ID NO:8, SEQ ID NO:9, SEQ ID NO:10, and SEQ ID NO:11, SEQ ID NO:12, SEQ ID NO:13, SEQ ID NO:14, SEQ ID NO:15, SEQ ID NO:16, or any functional fragment thereof. In some embodiments, the methods comprise an enzyme exhibiting endo-1,3(4)-beta-glucanase activity in combination with an enzyme exhibiting xylanase activity, each independently selected from the groups described above. In some embodiments, the methods comprise an enzyme having xylanase activity of SEQ ID NO:1 and an enzyme exhibiting endo-1,3(4)-β-glucanase activity of SEQ ID NO:7. In some embodiments, the methods comprise an enzyme having xylanase activity of SEQ ID NO:2 and an enzyme exhibiting endo-1,3(4)-β-glucanase activity of SEQ ID NO:8. In some embodiments, the methods comprise an enzyme having xylanase activity of SEQ ID NO:2 and an enzyme exhibiting endo-1,3(4)-β-glucanase activity of SEQ ID NO:8. In some embodiments, any of the above combination of an enzyme being an enzyme exhibiting endo-1,4-beta-xylanase activity with an enzyme exhibiting endo-1,3(4)-beta-glucanase activity may be combined with a ratio between the two enzymes of 1:1 per weight. In some embodiments, any of the above combination, comprise an enzyme exhibiting endo-1,3(4)-beta-glucanase activity having a minimum activity of 9000 U/g and an enzyme exhibiting endo-1,4-beta-xylanase activity having a minimum activity of 13000U/g.

In some embodiments the composition and methods according to the invention comprises the use of any suitable commercially available enzymes having xylanase activity and/or beta-glucanase activity such as UltraFlo L (available from Novozymes -beta glucanase with cellulase, xylanase side activities), UltraFlo XL (available from Novozymes -beta glucanase with xylanase and alpha amylase side activities), UltraFlo Max (available from Novozymes - beta glucanase and xylanase), Finizyme 250 L(available from Novozymes - beta glucanase with cellulase, xylanase side activities) Filtrase series(available from DSM-betaglucanase and xylanases).

In some embodiments, the cereal and one or more enzymes described herein (e.g., a beta glucanase and/or a xylanase) are combined at an effective concentration during one or more of the wetting of the cereal, and the combination is held at a temperature (e.g. of at least about 5°C and not more than about 30°C, preferably between about 10°C to about 20°C) for a period of time to hydrolyse at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % of the β-glucans and/or arabinoxylans in grain. In some embodiments, the cereal and one or more enzymes described herein (e.g., a beta glucanase and/or a xylanase) are combined to hydrolyze at least 50% of the β-glucans and/or arabinoxylans in grain. In some embodiments, the cereal and one or more enzymes described herein (e.g., a beta glucanase and/or a xylanase) are combined to hydrolyze at least 60% of the β-glucans and/or arabinoxylans in grain. In some embodiments, the cereal and one or more enzymes described herein (e.g., a beta glucanase and/or a xylanase) are combined to hydrolyze at least 70% of the β-glucans and/or arabinoxylans in grain. In some embodiments, the cereal and one or more enzymes described herein (e.g., a beta glucanase and/or a xylanase) are combined to hydrolyze at least 80% of the β-glucans and/or arabinoxylans in grain.

In some embodiments, at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % of the β-glucans and/or arabinoxylans in grain are broken down compared to a control malted cereal prepared without the one or more enzymes according to the methods described herein. In some embodiments, at least 10 % of the β-glucans and/or arabinoxylans in grain are broken down compared to a control malted cereal prepared without the one or more enzymes according to the methods described herein. In some embodiments, at least 50 % of the β-glucans and/or arabinoxylans in grain are broken down compared to a control malted cereal prepared without the one or more enzymes according to the methods described herein. In some embodiments, at least 60 % of the β-glucans and/or arabinoxylans in grain are broken down compared to a control malted cereal prepared without the one or more enzymes according to the methods described herein. In some embodiments, at least 70 % of the β-glucans and/or arabinoxylans in grain are broken down compared to a control malted cereal prepared without the one or more enzymes according to the methods described herein. In some embodiments, at least 80 % of the β-glucans and/or arabinoxylans in grain are broken down compared to a control malted cereal prepared without the one or more enzymes according to the methods described herein.

In some embodiments, at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % of the β-glucans in grain are broken down compared to a control malted cereal prepared without the one or more enzymes according to the methods described herein. In some embodiments, at least 10 % of the β-glucans in grain are broken down compared to a control malted cereal prepared without the one or more enzymes according to the methods described herein. In some embodiments, at least 50 % of the β-glucans in grain are broken down compared to a control malted cereal prepared without the one or more enzymes according to the methods described herein. In some embodiments, at least 60 % of the β-glucans in grain are broken down compared to a control malted cereal prepared without the one or more enzymes according to the methods described herein. In some embodiments, at least 80 % of the β-glucans in grain are broken down compared to a control malted cereal prepared without the one or more enzymes according to the methods described herein.

In some embodiments, the β-glucans in grain are broken down such that the concentration of high molecular weight β-glucan in the malted cereal is less than 150 mg/l, or less than 100 mg/l or less than 90 mg/l, or less than 80 mg/l, or less than 70 mg/l, or less than 60 mg/l, or less than 50 mg/l. In some embodiments, the β-glucans in grain are broken down such that the concentration of high molecular weight β-glucan in the malted cereal is 50 mg/l or less.

In some embodiments, at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % of the arabinoxylans in grain are broken down compared to a control malted cereal prepared without the one or more enzymes according to the methods described herein. In some embodiments, at least 10 % of the arabinoxylans in grain are broken down compared to a control malted cereal prepared without the one or more enzymes according to the methods described herein

In some embodiments, at least 50 % of the β-glucans in grain are broken down and at least 50 % of the arabinoxylans are broken down compared to a control malted cereal prepared without the one or more enzymes according to the methods described herein. In some embodiments, at least 60 % of the β-glucans in grain are broken down and at least 50 % of the arabinoxylans are broken down compared to a control malted cereal prepared without the one or more enzymes according to the methods described herein. In some embodiments, at least70 % of the β-glucans in grain are broken down and at least 50 % of the arabinoxylans are broken down compared to a control malted cereal prepared without the one or more enzymes according to the methods described herein. In some embodiments, at least 80 % of the β-glucans in grain are broken down and at least 50 % of the arabinoxylans are broken down compared to a control malted cereal prepared without the one or more enzymes according to the methods described herein.

In some embodiments, the arabinoxylans in grain are broken down such that the concentration of high molecular weight arabinoxylans in the malted cereal is less than 2400 mg/l, or less than 2200 mg/l, or less than 1900 mg/l, or less 1500 than mg/l, or less than 1000 mg/l, or less than 800 mg/l, or less than 700 mg/l or less than 600 mg/l, or less than 500 mg/l, or less than 100 mg/l, or less than 60 mg/l, or less than 50 mg/l. In some embodiments, the arabinoxylans in grain are broken down such that the concentration of high molecular weight arabinoxylans in the malted cereal is 2000 mg/l or less.

In some embodiments, the cereal and one or more enzymes described herein (e.g., a beta glucanase and/or a xylanase) are combined at an effective concentration during one or more of the wetting of the cereal at a specific temperature(s) for a period of time until the cereal has a moisture content of at least about 20 weight percent. In yet other embodiments, after the wetted cereal has attained increased moisture content and has started to germinate. In still other embodiments, the moistened cereal and the one or more enzymes are combined at an effective concentration during one or more of the wetting of the cereal for a period of time and temperature until the cereal has a moisture content of between about 20 to about 60 weight percent, or about 40 to 50 weight percent. In some embodiments the moistened cereal and the one or more enzymes are combined at an effective concentration during one or more of the wetting of the cereal for a period of time and temperature until the cereal has a moisture content of between about 40 to about 47 weight percent, and has germinated. In some embodiments, the total time of the steeping process is less compared to the time of a control malted cereal prepared without the one or more enzymes according to the methods described herein.

In still other embodiments, the moistened cereal and the one or more enzymes are combined at an effective concentration during one or more of the wetting of the cereal for a period of time and temperature until the cereal has a moisture content of between about 20 to about 60 weight percent, or about 40 to 50 weight percent, and the β-glucans in grain are broken down such that the concentration of high molecular weight β-glucan in the malted cereal is 50 mg/l or less. In some embodiments the moistened cereal and the one or more enzymes are combined at an effective concentration during one or more of the wetting of the cereal for a period of time and temperature until the cereal has a moisture content of between about 40 to about 47 weight percent, has germinated and the β-glucans in grain are broken down such that the concentration of high molecular weight β-glucan in the malted cereal is 50 mg/l or less. In some embodiments, the total time of the steeping process is less compared to the time of a control malted cereal prepared without the one or more enzymes according to the methods described herein.

In still other embodiments, the moistened cereal and the one or more enzymes are combined at an effective concentration during one or more of the wetting of the cereal for a period of time and temperature until the cereal has a moisture content of between about 20 to about 60 weight percent, or about 40 to 50 weight percent, and the arabinoxylans in grain are broken down such that the concentration of high molecular weight arabinoxylans in the malted cereal is 2000 mg/l or less. In some embodiments the moistened cereal and the one or more enzymes are combined at an effective concentration during one or more of the wetting of the cereal for a period of time and temperature until the cereal has a moisture content of between about 40 to about 47 weight percent, has germinated and the arabinoxylans in grain are broken down such that the concentration of high molecular weight arabinoxylans in the malted cereal is 2000 mg/l or less. In some embodiments the moistened cereal and the one or more enzymes are combined at an effective concentration during one or more of the wetting of the cereal for a period of time and temperature until the cereal has a moisture content of between about 40 to about 47 weight percent, has germinated and the arabinoxylans in grain are broken down such that the concentration of high molecular weight arabinoxylans in the malted cereal is 1000 mg/l or less. In some embodiments, the total time of the steeping process is less compared to the time of a control malted cereal prepared without the one or more enzymes according to the methods described herein.

In some embodiments, the total time for the steeping process is at least 5, 7, 9, 10, 11, 13, 15, 17, 19, 20, 21, 23, 25, 27, 29, 30, 31, 33, 35, 37, 39, 40, 41, 43, 45, 47, 49, 50, 51, 53, 55, 57, 59, 60, 61, 63, 65, 67, 69, 70, 71, 73, 75, 77, 79, 80, 81, 83, 85, 87, 89, 91, 93 or 95 % less than the time of a control malted cereal prepared without the one or more enzymes according to the methods described herein.

In some embodiments, the total time for the steeping process does not exceed 30 hours. In some embodiments, the total time for the steeping process does not exceed 25 hours. In some embodiments, the total time for the steeping process is about 10 hours to about 25 hours. In some embodiments, the total time for the steeping process is less than 25, 20, 15, or even less than 10 hours.

In some embodiments, the total amount of water used during the steeping process is at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % less than the water used for a control malted cereal prepared without the one or more enzymes according to the methods described herein. In some embodiments, the total amount of water used during the steeping process is at least 10 % less than the water used for a control malted cereal prepared without the one or more enzymes according to the methods described herein. In some embodiments, the total amount of water used during the steeping process is at least 20 % less than the water used for a control malted cereal prepared without the one or more enzymes according to the methods described herein. In some embodiments, the total amount of water used during the steeping process is at least 30 % less than the water used for a control malted cereal prepared without the one or more enzymes according to the methods described herein.

In some embodiments, the invention provides for improved water uptake by the grain. In some embodiments, the water uptake by the grain is improved by at least 10, 15, 20, 50, 70, 90 or 95% compared to a control malted cereal prepared without the one or more enzymes according to the methods described herein. In some embodiments, the water uptake by the grain is improved by at least 10%, or at least 20%.

In some embodiments, the cereal and one or more enzymes described herein (e.g., a beta glucanase and/or a xylanase) are combined at an effective concentration at the beginning of the first wetting of the cereal. In some embodiments, the cereal and one or more enzymes described herein (e.g., a beta glucanase and/or a xylanase) are combined at an effective concentration during the first wetting of the cereal. In some embodiments, the cereal and one or more enzymes described herein (e.g., a beta glucanase and/or a xylanase) are combined at an effective concentration during the first wetting of the cereal after a desired temperature have been reached. In some embodiments, the wetting stage involves spraying the cereal, where one or more enzymes as described herein are added one or more times, or constantly, during the spraying.

In some embodiments, the cereal and one or more enzymes described herein (e.g., a beta glucanase and/or a xylanase) are combined at an effective concentration 1, 2, 3 or 4 hours after the beginning of the first wetting of the cereal. In some embodiments, the cereal and one or more enzymes described herein (e.g., a beta glucanase and/or a xylanase) are combined during the last hour of the first wetting of the cereal.

In some embodiments, the cereal and one or more enzymes described herein (e.g., a beta glucanase and/or a xylanase) are combined at an effective concentration at the beginning of the second wetting of the cereal. In some embodiments, the cereal and one or more enzymes described herein (e.g., a beta glucanase and/or a xylanase) are combined at an effective concentration during the second wetting of the cereal. In some embodiments, the cereal and one or more enzymes described herein (e.g., a beta glucanase and/or a xylanase) are combined at an effective concentration during the second wetting of the cereal after a desired temperature have been reached. In some embodiments, the wetting stage involves spraying the cereal, where one or more enzymes as described herein are added one or more times, or constantly, during the spraying.

In some embodiments, the cereal and one or more enzymes described herein (e.g., a beta glucanase and/or a xylanase) are combined at an effective concentration 1, 2, 3 or 4 hours after the beginning of the second wetting of the cereal. In some embodiments, the cereal and one or more enzymes described herein (e.g., a beta glucanase and/or a xylanase) are combined during the last hour of the second wetting of the cereal.

In some embodiments, the cereal and one or more enzymes described herein (e.g., a beta glucanase and/or a xylanase) are combined at an effective concentration at the beginning of the third wetting of the cereal. In some embodiments, the cereal and one or more enzymes described herein (e.g., a beta glucanase and/or a xylanase) are combined at an effective concentration during the third wetting of the cereal. In some embodiments, the cereal and one or more enzymes described herein (e.g., a beta glucanase and/or a xylanase) are combined at an effective concentration during the third wetting of the cereal after a desired temperature have been reached. In some embodiments, the wetting stage involves spraying the cereal, where one or more enzymes as described herein are added one or more times, or constantly, during the spraying.

In some embodiments, the cereal and one or more enzymes described herein (e.g., a beta glucanase and/or a xylanase) are combined at an effective concentration 1, 2, 3 or 4 hours after the beginning of the third wetting of the cereal. In some embodiments, the cereal and one or more enzymes described herein (e.g., a beta glucanase and/or a xylanase) are combined during the last hour of the third wetting of the cereal.

In some embodiments, the cereal and one or more enzymes described herein (e.g., a beta glucanase and/or a xylanase) are combined at an effective concentration at the beginning of each of the wetting stages of the cereal. In some embodiments, the cereal and one or more enzymes described herein (e.g., a beta glucanase and/or a xylanase) are combined at an effective concentration during all the wetting stages of the cereal. In some embodiments, the cereal and one or more enzymes described herein (e.g., a beta glucanase and/or a xylanase) are combined at an effective concentration during all the wetting stages of the cereal after a desired temperature have been reached. In some embodiments, the one or more wetting stages involve spraying the cereal, where one or more enzymes as described herein are added one or more times, or constantly, during the spraying.

In some embodiments, the present invention provides malted cereals, obtained according to the process of the invention, which present improved properties. For instance, the malted cereals produced by the methods described herein have a lower high molecular weight fraction of beta-glucan and/or arabinoxylans content, and/or a higher enzyme activity such as, for example, beta-glucanase or xylanase activity than a control malted cereal prepared without the one or more enzymes according to the methods described herein. This allows for a better processability of the malt, e.g., in wort and beer production by potentially having increased rates of mash separation and beer filtration. In some embodiments, the malted cereals produced by the methods described herein have a concentration of high molecular weight β-glucan of 100 mg/l, 90 mg/l, 80 mg/l, 70 mg/l, 60 mg/l, 50 mg/l or less. In some embodiments, the malted cereals produced by the methods described herein have a concentration of high molecular weight arabinoxylans of 2500 mg/l, 2200 mg/l, 2100 mg/l, 1900 mg/l, 1500 mg/l, 1000 mg/1800 mg/l, 700 mg/l, 600 mg/l, 500 mg/l, 100 mg/l, 50 mg/l or less. In some embodiments, malted cereals produced by the methods described herein have a beta-glucanase activity and/or xylanase activity of about 10 U/kg - 100 U/kg, or about 30 U/kg - 100 U/kg, or about 50 U/kg - 100 U/kg are utilized. In some embodiments about 100 U/kg - 1000 U/kg.

In some embodiments, an object of the present invention concerns the use of the malted cereals according to the invention for the preparation of food and beverages. The invention is also related to these improved beverages. The improved malted cereals according to the invention could also be used in other biotechnological processes well known by a person of ordinary skill in the art, in which in most cases advantage is taken of their improved quality.

In some embodiments, the methods describes herein provide for a malted cereal with a decrease amount of high molecular weight fraction of β-glucans and/or arabinoxylans. In some embodiments, the total amount of high molecular weight β-glucans and/or arabinoxylans is at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % less than the amount of high molecular weight β-glucans and/or arabinoxylans in a control malted cereal prepared without the one or more enzymes according to the methods described herein.

In some embodiments, the total amount of high molecular weight β-glucans is at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % less than the amount of β-glucans in a control malted cereal prepared without the one or more enzymes according to the methods described herein. In some embodiments, the total amount of high molecular weight β-glucans is at least 50 % less than the amount of high molecular weight β-glucans in a control malted cereal prepared without the one or more enzymes according to the methods described herein. In some embodiments, the total amount of high molecular weight β-glucans is at least 60 % less than the amount of high molecular weight β-glucans in a control malted cereal prepared without the one or more enzymes according to the methods described herein. In some embodiments, the total amount of high molecular weight β-glucans is at least 70 % less than the amount of high molecular weight β-glucans in a control malted cereal prepared without the one or more enzymes according to the methods described herein. In some embodiments, the total amount of high molecular weight β-glucans is at least 80 % less than the amount of high molecular weight β-glucans in a control malted cereal prepared without the one or more enzymes according to the methods described herein.

In some embodiments, the total amount of arabinoxylans is at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % less than the amount of arabinoxylans in a control malted cereal prepared without the one or more enzymes according to the methods described herein. In some embodiments, the total amount of high molecular weight arabinoxylans is at least 50 % less than the amount of high molecular weight arabinoxylans in a control malted cereal prepared without the one or more enzymes according to the methods described herein.

In some embodiments, the total amount of high molecular weight arabinoxylans is at least 50 % less than the amount of high molecular weight arabinoxylans and the total amount of high molecular weight β-glucans is at least 80 % less than in a control malted cereal prepared without the one or more enzymes according to the methods described herein.

In some embodiments, the methods described herein provide for a malted cereal with lower viscosity, when extracted. In some embodiments, the viscosity of the malted cereal is at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % lower than the viscosity of a malted cereal prepared without the one or more enzymes according to the invention.

In some embodiments, the malted cereals described herein provide for the production of an extract with lower viscosity. In some embodiments, the viscosity of the extract is at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % lower than the viscosity of a extract prepared with a control malted cereal prepared without the one or more enzymes according to the invention.

In some embodiments, the process of the invention may increase the enzymatic activity of the malted cereal. In some embodiments, the enzymatic activity is selected from the group consisting of β-glucanase activity, xylanase activity, amylase activity, protease activity, naturally occurring enzyme activity in the cereal and combinations thereof.

In some embodiments, the process of the invention may increase a β-glucanase activity and/or a xylanase activity of a malted cereal by a factor of at least about 2 as compared to a control malted cereal prepared without the one or more enzymes according to the invention. In some embodiments, a β-glucanase activity and/or a xylanase activity of a malted cereal is at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % higher than a control malted cereal prepared without the one or more enzymes according to the invention. In some embodiments, malted cereals produced by the methods described herein have a beta-glucanase activity and/or xylanase activity of about 10 U/kg - 100 U/kg, or about 30 U/kg - 100 U/kg, or about 50 U/kg - 100 U/kg are utilized. In some embodiments about 100 U/kg - 1000 U/kg.

Thus is some embodiments, the cereal and one or more enzymes described herein (e.g., a beta glucanase and/or a xylanase) are combined at an effective concentration during at least one of the wetting of the cereal for a period of time and at a temperature to obtain an increase in enzymatic activity of the malted cereal. In some embodiments, the enzymatic activity is selected from the group consisting of β-glucanase activity, xylanase activity, amylase activity, protease activity, naturally occurring enzyme activity in the cereal and combinations thereof.

In some embodiments the one or more enzymes described herein are added at the final stages of the malting process or after the malting process is completed to further increase the enzymatic activity of the malt.

In some embodiments, the methods described herein provide for a malted cereal with a lower potential for off flavor formation, such as off flavor formation related to arabinoxylan breakdown.

In some embodiments the methods described herein provide for a malted cereal with a decreased risk of filter bed collapse, such as at lautering.

In some embodiments the total pressure built up is reduced to a value of less than 470 mm WC, such as less than 450 mm WC, such as less than 430 mm WC, such as less than 410 mm WC, such as less than 390 mm WC, such as less than 370 mm WC, such as less than 350 mm WC, such as less than 330 mm WC, such as less than 310 mm WC, such as less than 300 mm WC, such as less than 290 mm WC, when a malted cereal according to the present invention is used prior to the mash separation in a brewing application.

In some embodiments the total pressure built up is reduced by at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93 or 95 % compared to the use of a negative control without said malted cereal; when used prior to the mash separation in a brewing application.

In some embodiments the mash separation as measured by volume wort collected after 5 min of separation relative to a control malted cereal is increased to above 1.5, such as above 1.6, such as above 1.7, such as above 1.8, such as above 1.9, such as above 2.0, such as above 2.1, such as above 2.2, such as above 2.3, such as above 2.4, such as above 2.5, when the composition according to the invention is used in a brewing application prior to the mash separation.

In some embodiments the mash separation as measured by volume wort collected after 5 min of filtration is increased at least 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 110, 120, 130, 140, 150, 160, 170, 180, 190, 200, 210, 220, 230, 240, 250, 260, 270, 280, 290, or 300 % as compared to the use of a control malted cereal.

The process of malting is well known in the art and the specific conditions vary depending on the type of cereal used, the final specifications needed for the malted cereals and the specific malting facilities. The methods described herein can be applied to any malting process known in the art and can be used in any malting facility. It is to be understood that certain parameters of the compositions and methods described herein may be adjusted depending to the properties needed for the malt and/or the specific malting facilities to obtain such optimal properties in the malted cereal, e.g., depending on the cereal varieties and the downstream uses.

### EXAMPLES

The present disclosure is described in further detail in the following examples, which are not in any way intended to limit the scope of the disclosure as claimed. The following examples are offered to illustrate, but not to limit the claimed disclosure.

### Example 1 - Assays to Measure Enzyme Activity

### DNS Cellulase activity method (DNS CMC method)

### Systematic Name: 1,4-(1,3;1,4)- beta-D-glucan 4-glucanohydrolase

### IUB Number: EC 3.2.1.4

### Principle

The assay of cellulase is based on the enzymatic endo-hydrolysis of the 1,4-β-D-glucosidic bonds in carboxymethylcellulose (CMC), a β-1,4-glucan. The products of the reaction (β-1,4 glucan oligosaccharides) is determined colorimetrically by measuring the resulting increase in reducing groups using a 3,5 dinitrosalicylic acid reagent. Enzyme activity is calculated from the relationship between the concentration of reducing groups, as glucose equivalents, and absorbance at 540nm.

The assay is carried out at pH 5.0, but it can be performed at different pH values for the additional characterization and specification of enzymes.

### Unit definition

One unit of cellulase activity is defined as the amount of enzyme which produces 1 µmole glucose equivalents per minute under the conditions of the assay (pH 5.0 (or as specified) and 50 °C).

### Materials

Carboxymethylcellulose. Supplier: Megazyme Ltd. Product no.: CM-Cellulose 4M; D-Glucose 'AnalaR'. Supplier: Merck Ltd (BDH). Product no.: 10117. M.W.: 180.16; Sodium acetate anhydrous 'AnalaR'. Supplier: Merck Ltd (BDH). Product no.: 10236. M.W.: 82.03; Acetic acid ("glacial") 'AnalaR'. Supplier: Merck Ltd (BDH). Product no.: 10001. M.W.: 60.05; 3,5-Dinitrosalicylic acid GPR (3,5-dinitro-2-hydroxybenzoic acid). Supplier: Merck Ltd (BDH). Product no.: 28235; Sodium hydroxide pellets 'AnalaR'. Supplier: Merck Ltd (BDH). Product no.: 10252. M.W.: 40.00; Potassium sodium (+)-tartrate 'AnalaR'. Supplier: Merck Ltd (BDH). Product no.: 10219. M.W.: 282.22; 1.5 %(w/v solution) Carboxymethylcellulose (CMC) solution in 0.1M sodium acetate buffer, pH 5.0 (substrate solution); 3,5-Dinitrosalicylic acid (DNS) solution. 20g/L of DNS in buffer containing 32g/L sodium hydroxide pellets, and 600 g/L potassium sodium (+)-tartrate; Glucose standard solution (0.50 mg/ml)

### Procedure

The enzyme composition is diluted into samples and a glucose standard curve can be made using glucose concentrations of 0, 0.125, 0.25, 0.375, and 0.5 mg/ml.

0.25 ml of enzyme solution is mixed with 1.75 ml of the substrate solution (1.5% w/v) at 50 °C and the reaction is stopped after 10 min by addition of DNS solution. This is followed by heating to 95 °C for 5 minutes.

The optical density is measured at 540 nm (OD540nm) of the different samples.

### Calculation

The enzyme activity is determined from the standard curve.

The activity is calculated as described in PCT publication number WO2013/037933.

### Laminarinase (DNS laminarin method)

### Principle

The reaction, catalysed by laminarinase, involves the endohydrolysis of 1,3- glucosidic bonds in 1,3-β-D-glucans. Substrates include laminarin, paramylon and pachyman. The products of the reaction (β-1,3-glucan oligosaccharides) are determined colourimetrically by measuring the resulting increase in reducing groups using a 3,5 dinitrosalicylic acid reagent. Enzyme activity is calculated from the relationship between the concentration of reducing groups, as glucose equivalents, and absorbance at 540nm.

The assay can be carried out at pH 5.0 and 50 °C, it can also be performed at different values of pH and temperature for the additional characterization and specification of enzymes.

### Unit definition

One unit of laminarinase activity is defined as the amount of enzyme which produces 1 µmole glucose equivalents per minute under the conditions of the assay (pH 5.0 and 50 °C (or as specified)).

### Materials

See materials given above for the Cellulase activity assay.

Laminarin (from Laminaria digitata). Supplier: Sigma-Aldrich Co. Ltd. Product no.: L 9634; 1.00 %(w/v solution) Laminarin solution (substrate solution 0.1M sodium acetate buffer, pH 5.0); 1.75 ml laminarin solution is mixed with 0.25 ml diluted enzyme solution at 50 °C for 10 minutes and the reaction stopped by addition of 2 ml DNS solution.

Standard curve can be made using 0, 0.125, 0.25, 0.5 and 0.75 mg/ml glucose solution.

Optical density is measured at 540 nm (OD540nm).

### Calculation

The activity is calculated as described in PCT publication number WO2013/037933.

### Arabinase Assay

### Principle

The assay of Arabinase activity is based on colorimetrically determination by measuring the resulting increase in reducing groups using a 3,5 dinitrosalicylic acid reagent. Enzyme activity is calculated from the relationship between the concentration of reducing groups, as arabinose equivalents, and absorbance at 540nm.

The assay can be carried out at pH 3.5, it can also be performed at different pH values for the additional characterization and specification of enzymes.

### Unit definition

One unit of arabinase (Arabinanase (endo-1,5-alpha-L-arabinanase)) activity is defined as the amount of enzyme which produces 1 µmole arabinose equivalents per minute under the conditions of the assay (pH 3.5 (or as specified) and 50 °C).

### Materials

Megazyme Sugar Beet Arabinan; Arabinose Sigma A3131 M.W.: 150.1;Sodium acetate anhydrous 'AnalaR'. Supplier: Merck Ltd (BDH). Product no.: 10236. M.W.: 82.03; Acetic acid ("glacial") 'AnalaR'. Supplier: Merck Ltd (BDH). Product no.: 10001. M.W.: 60.05; 3,5-Dinitrosalicylic acid GPR (3,5-dinitro-2-hydroxybenzoic acid). Supplier: Merck Ltd (BDH). Product no.: 28235; Sodium hydroxide pellets 'AnalaR'. Supplier: Merck Ltd (BDH). Product no.: 10252. M.W.: 40.00; Potassium sodium (+)-tartrate 'AnalaR'. Supplier: Merck Ltd (BDH). Product no.: 10219. M.W.: 282.22; 1.5 %(w/v solution) Arabinan solution in 0.1M sodium acetate buffer, pH 3.5 (substrate solution); 3,5-Dinitrosalicylic acid (DNS) solution. 20g/L of DNS in buffer containing 32g/L sodium hydroxide pellets, and 600 g/L potassium sodium (+)-tartrate; Arabinase standard solution (0.50 mg/ml)

### Procedure

The enzyme composition is diluted into samples and an arabinase standard curve is made using arabinase concentrations of 0, 0.125, 0.25, 0.375, and 0.5 mg/ml.

0.25 ml of enzyme solution is mixed with 1.75 ml of the substrate solution (1.5% w/v) at 50 °C and the reaction is stopped after 10 min by addition of DNS solution. This is then followed by heating to 95 °C for 5 minutes.

The optical density is measured at 540 nm (OD540nm) of the different samples.

### Calculation

The enzyme activity is determined from the standard curve.

The activity is calculated as described in PCT publication number WO2013/037933.

### Xylanase activity assay

Samples, to obtain approx. OD540 = 0.25 - 0.30 in this assay and xylose standards (0, 0.125, 0.250, 0.375 and 0.500 mg/ml distilled water) are prepared in distilled water. At time t=0 minutes, 1.75 ml soluble wheat arabinoxylan (0.5% wheat arabinoxylan (PWAXYH, Megazyme, Bray, Ireland)) in 0.1M sodium acetate/acetic acid, pH 5) is placed in a test tube at 50°C. At time t=5 minutes, 250µl enzyme solution is added to the substrate at 50°C followed by mixing. Distilled water is used as blank. At time t=15 minutes, 2ml DNS solution (1% 3,5-Dinitrosalicylic acid (DNS), 1.6% sodium hydroxide, 30% potassium sodium tartrate in distilled water) is added to the enzyme-substrate solution and 2.0 ml standard solution. Samples, blanks and standards added DNS are placed in a boiling water bath (95°C) for 5 minutes. Hereafter samples, blanks and standards are cooled by placing them in a 25°C water bath for 20 minutes. The optical density of all samples are read at OD540 using a spectrophotometer. Based on the dilution of the samples, the amount of sample used and the standards, the xylanase activity of the sample can be calculated.

One Unit of endo-1,4-beta-xylanase activity is defined as the amount of enzyme which produces 1 µmole xylose equivalents per minute under the conditions mentioned above

### Glucanase activity assay

Samples, to obtain OD540 within the standard curve in this assay and glucose standards (0; 0.125; 0.250; 0.500; and 0.750 mg/ml distilled water) are prepared in distilled water. At time t=0 minutes, 1,75 ml barley beta-glucan (1.5% barley beta-glucan (BETA-BGBM, Megazyme, Bray, Ireland)) in 1M sodium acetate/acetic acid, pH 5) is placed in a test tube at 50°C. At time t=5 minutes, 250µl enzyme solution is added to the substrate at 50°C followed by mixing. Distilled water is used as blank. At time t=15 minutes, 2ml DNS solution (1% 3,5-Dinitrosalicylic acid (DNS), 1,6% sodium hydroxide, 30% potassium sodium tartrate in distilled water) is added to the enzyme-substrate solution and 2.0 ml standard solution. Samples, blanks and standards added DNS are placed in a boiling water bath (95°C) for 15 minutes. Hereafter samples, blanks and standards are cooled by placing them in a 25°C water bath for 20 minutes. The Optical density of all samples are read at OD540 using a spectrophotometer. Based on the dilution of the samples, the amount of sample used and the standards, the glucanase activity of the sample can be calculated.

One unit of endo-1,3(4)-β-glucanase activity is defined as the amount of enzyme which produces 1 µmole glucose equivalents per minute under the conditions of the assay (pH 5.0 (or as specified) and 50 °C).

### Arabinofuranosidase Assay

The reaction, catalysed by α-N-arabinofuranosidase, involves the hydrolysis of the terminal bond, at the non-reducing α-L-arabinofuranoside residue, of α-L-arabinosides. The enzyme acts on α-L-arabinofuranosides, α-L-arabinans containing (1,3)- and/or (1,5)-linkages, arabinoxylans and arabinogalactans.

The assay of α-N-arabinofuranosidase is based upon the enzymatic hydrolysis of p-nitrophenyl α-L-arabinofuranoside. The assay is a "two-point", rather than a "continuous monitoring", method. The calculation of enzyme activity is based on measurements taken only at the beginning and end of the incubation period. A product of the reaction, p-nitrophenol is determined colourimetrically (after pH adjustment). Enzyme activity is calculated from the relationship between the concentration of p-nitrophenol and absorbance at 400nm.

### Preparation of diluted enzyme solution and Procedure

The enzyme solution is prepared and the assay is performed as described in PCT publication number WO2013/037933.

### Calculation

The activity is calculated as described in PCT publication number WO2013/037933.

### Cellobiohydrolase assay

### Principle

The reaction, catalyzed by cellobiohydrolase, involves the hydrolysis of 1,4-β-D-glucosidic linkages in cellulose and cellotetraose, releasing cellobiose from the non-reducing ends of the chains.

The assay of cellobiohydrolase is based on the enzymatic hydrolysis of p-nitrophenyl β -D-cellobiopyranoside. The product of the reaction, p-nitrophenol is determined colorimetrically (after pH adjustment). Enzyme activity is calculated from the relationship between the concentration of p-nitrophenol and absorbance at 400nm.

The assay is operated within the linear defined range of ΔOD540nm TEST (T) = 0.400-0.800.

### Procedure

The assay is performed as described in PCT publication number WO2013/037933.

### Calculation

The activity is calculated as described in PCT publication number WO2013/037933.

### Example 2

A Joe White micro-malting system can be used to test multiple samples of different cereals from different varieties (e.g., barley: Sebastian, Tipple, Prestige, and Bellini), using different enzymes concentrations at different time points. This micro-malting system allows for steeping, germination, and kilning of samples in one unit while providing uniformity of malting conditions for each batch. Each sample is placed in one compartment of the micro-malting system. A sample of 0.5 kilogram of barley is added to each of the compartments in the micro-malting unit for malting. A mixture of enzymes containing an endo-1,3(4)-beta-glucanase and an endo-1,4-beta-xylanase is added at different times during the steeping process at the different concentrations 0.01 - 100 mg enzyme protein per kilogram. The table below describes experimental conditions.

| **Compartment/Sample** | **Barley Variety** | **Experimental Conditions** |
|---|---|---|
| 1 | Sebastian | No Enzymes |
| 2 | Sebastian | Enzymes added during the first wet stage |
| 3 | Sebastian | Enzymes added during the first and second wet stages |
| 4 | Sebastian | Enzymes added during all wet stages |
| 5 | Tipple | No Enzymes |
| 6 | Tipple | Enzymes added during the first wet stage |
| 7 | Tipple | Enzymes added during the first and second wet stages |
| 8 | Tipple | Enzymes added during all wet stages |
| 9 | Prestige | No Enzymes |
| 10 | Prestige | Enzymes added during the first wet stage |
| 11 | Prestige | Enzymes added during the first and second wet stages |
| 12 | Prestige | Enzymes added during all wet stages |
| 13 | Bellini | No Enzymes |
| 14 | Bellini | Enzymes added during the first wet stage |
| 15 | Bellini | Enzymes added during the first and second wet stages |
| 16 | Bellini | Enzymes added during all wet stages |

Three steeping cycles are used for all barley samples.

The barley can be steeped using different experimental conditions:
(a) the barley is steeped with the first water immersion for the time necessary for the grain to reach 40-45% moisture at a temperature of 15°C. Following the water immersion, the barley is subjected to air ventilation for 12 hours at a temperature of 15°C. The second immersion is for the time necessary for the grain to reach 40-45% moisture, which is then followed by air ventilation for 10 hours at 15°C. The third steeping is for 2 hours at 15°C. After steeping is completed, germination begins and the grains can be allowed to germinate for 5 days. The time necessary for each of the varieties to reach the desired levels of moisture is recorded.
(b) the barley is steeped with the first water immersion for 6 hours at a temperature of 15°C with a total water to air dry barley ratio of 3:1 for samples with no enzymes, 2:1 for samples in which the enzymes are added during the first wet stage, 1:1 for samples in which enzymes are added during the first and second wet stages; and 0.8:1 for samples in which enzymes are added during all wet stages. Following the water immersion, the barley is subjected to air ventilation for 12 hours at a temperature of 15°C. The second immersion is for 5 hours, followed by air ventilation for 0 hours at 15°C. The third immersion is for 2 hours at 15°C. After steeping is completed, the varieties can be germinated for 5 days.

Conditions (a) and (b) can be repeated using different enzymes concentrations, to determine differences in water consumption and/or germination time.

The germinating barley is subjected to humidified air at a temperature of 15°C.

After 5 days of germination, the barley in each compartment is kilned with a standard kilning program. Malt yield is measured in 1,000 kernel weight for each sample from each of the 16 compartments.

The amount of beta-glucans and arabinoxylans present in the germinating barley and final malt can be measured by any suitable methods known in the art. For example the amount of beta-glucans present in the germinating barley and final malt can be measured by the methods described in Journal of the Institute of Brewing Volume 91, Issue 5, pages 285-295, September-October 1985.

The glucanase and/or xylanase activity present in the malted cereal can be measured as described above.

Six quality parameters are measured on each malt sample, i.e. extract yield (%), total malt protein (%), soluble malt protein (%), Kolbach Index, apparent final attenuation (%) and viscosity (cp). The analytical procedures are used according to those recommended by the European Brewery Convention.

### Example 3

### Micromalting and malting quality analysis

Micromalting and subsequent analyses can be performed with two-rowed spring barley Troubadour from different locations. Troubadour, bred in The Netherlands and released in Spain in 1983, is very high-yielding under Mediterranean environments. However, its malting quality is fairly poor, giving low extract yields. It has been reported that the low yield could be correlated with the content of beta-glucan in the grain.

A micromalting plant that allows the processing of 32 samples of 200 g each per batch can be used. The micromalting procedure is as follows:
- Steeping time: 57 h (41 h under water and 16 h without water)
- Steeping temperature: 15 °C
- Germination time: 5 days
- Germination temperature: 15°C
- Air supply to germinating barley: 50 ml/min
- Drying time: 17 h
- Drying temperature: 50°C
- Kilning time: 2.5 h
- Kilning temperature: 70°C

Steeping is performed in the presence or absence of a mixture of enzymes containing an endo-1,3(4)-beta-glucanase and an endo-1,4-beta-xylanase at different times during the steeping process and at the different concentrations ranging from 0.01 - 100 mg enzyme protein per kilogram.

Six quality parameters are measured on each malt sample, i.e. extract yield (%), total malt protein (%), soluble malt protein (%), Kolbach Index, apparent final attenuation (%) and viscosity (cp). The analytical procedures are used according to those recommended by the European Brewery Convention.

Additionally, the total protein content, the beta-glucan and arabinoxylan content of barley and the beta-glucanase and xylanase activity of malt are determined as described above. While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### Sequences:

AtuXyn3, Aspergillus tubigensis (SEQ ID NO:1), 302 aa:
QASVSIDTKFKAHGKKYLGNIGDQYTLTKNSKTPAIIKADFGALTPENSMKWDATE
PSRGQFSFSGSDYLVNFAQSNNKLIRGHTLVWHSQLPSWVQAITDKNTLIEVMKNH
ITTVMQHYKGKIYAWDVVNEIFNEDGSLRDSVFYQVIGEDYVRIAFETARAADPNA
KLYINDYNLDSASYPKLTGMVSHVKKWIEAGIPIDGIGSQTHLSAGGGAGISGALNA
LAGAGTKEIAVTELDIAGASSTDYVEVVEACLDQPKCIGITVWGVADPDSWRSSSTP
LLFDSNYNPKPAYTAIANAL

TerXyn1, Geosmithia emersonii (Taleromyces emersonii) (SEQ ID NO:2):
AGLNTAAKAIGLKYFGTATDNPELSDTAYETQLNNTQDFGQLTPANSMKWDATEP
EQNVFTFSAGDQIANLAKANGQMLRCHNLVWYNQLPSWVTSGSWTNETLLAAMK
NHITNVVTHYKGQCYAWDVVNEALNDDGTYRSNVFYQYIGEAYIPIAFATAAAAD
PNAKLYYNDYNIEYPGAKATAAQNLVKLVQSYGARIDGVGLQSHFIVGETPSTSSQ
QQNMAAFTALGVEVAITELDIRMQLPETEALLTQQATDYQSTVQACANTKGCVGIT
VWDWTDKYSWVPSTFSGYGDACPWDANYQKKPAYEGILTGLGQTVTSTTYIISPT
TSVGTGTTTSSGGSGGTTGVAQHWEQCGGLGWTGPTVCASGYTCTVINEYYSQCL

AtuXyn4, Aspergillus tubigensis (SEQ ID NO:3):
EPIEPRQASVSIDTKFKAHGKKYLGNIGDQYTLTKNSKTPAIIKADFGALTPENSMK
WDATEPSRGQFSFSGSDYLVNFAQSNNKLIRGHTLVWHSQLPSWVQSITDKNTLIE
VMKNHITTVMQHYKGKIYAWDVVNEIFNEDGSLRDSVFYKVIGEDYVRIAFETAR
AADPNAKLYINDYNLDSASYPKLTGMVSHVKKWIAAGIPIDGIGSQTHLSAGGGAG
ISGALNALAGAGTKEIAVTELDIAGASSTDYVEVVEACLNQPKCIGITVWGVADPDS
WRSSSTPLLFDSNYNPKPAYTAIANAL

AacXyn2, Aspergillus aculeatus (SEQ ID NO:4):
MVGLLSITAALAATVLPNIVSAVGLDQAAVAKGLQYFGTATDNPELTDIPYVTQLN
NTADFGQITPGNSMKWDATEPSQGTFTFTKGDVIADLAEGNGQYLRCHTLVWYNQ
LPSWVTSGTWTNATLTAALKNHITNVVSHYKGKCLHWDVVNEALNDDGTYRTNI
FYTTIGEAYIPIAFAAAAAADPDAKLFYNDYNLEYGGAKAASARAIVQLVKNAGAK
IDGVGLQAHFSVGTVPSTSSLVSVLQSFTALGVEVAYTEADVRILLPTTATTLAQQS

SDFQALVQSCVQTTGCVGFTIWDWTDKYSWVPSTFSGYGAALPWDENLVKKPAY
NGLLAGMGVTVTTTTTTTATATGKTTTTTTGATSTGTTAAHWGQCGGLNWSGPT
ACATGYTCTYVNDYYSQCL

TreXyn3, Trichoderma reesei (SEQ ID NO:5) :
MKANVILCLLAPLVAALPTETIHLDPELAALRANLTERTADLWDRQASQSIDQLIKR
KGKLYFGTATDRGLLQREKNAAIIQADLGQVTPENSMKWQSLENNQGQLNWGDA
DYLVNFAQQNGKSIRGHTLIWHSQLPAWVNNINNADTLRQVIRTHVSTVVGRYKG
KIRAWDVVNEIFNEDGTLRSSVFSRLLGEEFVSIAFRAARDADPSARLYINDYNLDR
ANYGKVNGLKTYVSKWISQGVPIDGIGSQSHLSGGGGSGTLGALQQLATVPVTELA
ITELDIQGAPTTDYTQVVQACLSVSKCVGITVWGISDKDSWRASTNPLLFDANFNPK
PAYNSIVGILQ

TreXyn5, Trichoderma reesei (SEQ ID NO:6):
QCIQPGTGYNNGYFYSYWNDGHGGVTYCNGPGGQFSVNWSNSGNFVGGKGWQP
GTKNRVINFSGSYNPNGNSYLSVYGWSRNPLIEYYIVENFGTYNPSTGATKLGEVTS
DGSVYDIYRTQRVNQPSIIGTATFYQYWSVRRNHRSSGSVNTANHFNAWAQQGLT
LGTMDYQIVAVEGYFSSGSASITVSD

BsuGluS, Bacillus subtilis (SEQ ID NO:7), 214 aa:
QTGGSFFDPFNGYNSGFWQKADGYSNGNMFNCTWRANNVSMTSLGEMRLALTSP
AYNKFDCGENRSVQTYGYGLYEVRMKPAKNTGIVSSFFTYTGPTDGTPWDEIDIEF
LGKDTTKVQFNYYTNGAGNHEKIVDLGFDAANAYHTYAFDWQPNSIKWYVDGQL
KHTATNQIPTTPGKIMMNLWNGTGVDEWLGSYNGVNPLYAHYDWVRYTKK

TerGlu1, Geosmithia emersonii (Taleromyces emersonii) (SEQ ID NO:8):
APVKEKGIKKRASPFQWFGSNESGAEFGNNNIPGVEGTDYTFPNTSAIQILIDQGMNI
FRVPFLMERMVPNQMTGPVDSAYFQGYSQVINYITSHGASAVIDPHNFGRYYNNIIS
SPSDFQTFWHTIASNFADNDNVIFDTNNEYHDMDESLVVQLNQAAIDGIRAAGATS
QYIFVEGNSWTGAWTWTQVNDAMANLTDPQNKIVYEMHQYLDSDGSGTSDQCV
NSTIGQDRVESATAWLKQNGKKAILGEYAGGANSVCETAVTGMLDYLANNTDVW
TGAIWWAAGPWWGDYIFSMEPPSGIAYEQVLPLLQPYL

BsuGlu103FULL, Bacillus subtilis (SEQ ID NO:9):
DDYSVVEEHGQLSISNGELVNERGEQVQLKGMSSHGLQWYGQFVNYESMKWLRD
DWGITVFRAAMYTSSGGYIDDPSVKEKVKETVEAAIDLGIYVIIDWHILSDNDPNIY
KEEAKDFFDEMSELYGDYPNVIYEIANEPNGSDVTWDNQIKPYAEEVIPVIRDNDPN
NIVIVGTGTWSQDVHHAADNQLADPNVMYAFHFYAGTHGQNLRDQVDYALDQG
AAIFVSEWGTSAATGDGGVFLDEAQVWIDFMDERNLSWANWSLTHKDESSAALM
PGANPTGGWTEAELSPSGTFVREKIRESASIPPSDPTPPSDPGEPDPGEPDPTPPSDPG
EYPAWDSNQIYTNEIVYHNGQLWQAKWWTQNQEPGDPYGPWEPLKSDPDSGEPD
PTPPSDPGEYPAWDSNQIYTNEIVYHNGQLWQAKWWTQNQEPGDPYGPWEPLN

TreGlu2, Trichoderma reesei (SEQ ID NO:10):
QQTVWGQCGGIGWSGPTNCAPGSACSTLNPYYAQCIPGATTITTSTRPPSGPTTTTR
ATSTSSSTPPTSSGVRFAGVNIAGFDFGCTTDGTCVTSKVYPPLKNFTGSNNYPDGIG
QMQHFVNDDGMTIFRLPVGWQYLVNNNLGGNLDSTSISKYDQLVQGCLSLGAYCI
VDIHNYARWNGGIIGQGGPTNAQFTSLWSQLASKYASQSRVWFGIMNEPHDVNINT
WAATVQEVVTAIRNAGATSQFISLPGNDWQSAGAFISDGSAAALSQVTNPDGSTTN
LIFDVHKYLDSDNSGTHAECTTNNIDGAFSPLATWLRQNNRQAILTETGGGNVQSCI
QDMCQQIQYLNQNSDVYLGYVGWGAGSFDSTYVLTETPTGSGNSWTDTSLVSSCL
ARK

TreGlu3, Trichoderma reesei (SEQ ID NO:11):
QTSCDQWATFTGNGYTVSNNLWGASAGSGFGCVTAVSLSGGASWHADWQWSGG
QNNVKSYQNSQIAIPQKRTVNSISSMPTTASWSYSGSNIRANVAYDLFTAANPNHVT
YSGDYELMIWLGKYGDIGPIGSSQGTVNVGGQSWTLYYGYNGAMQVYSFVAQTN
TTNYSGDVKNFFNYLRDNKGYNAAGQYVLSYQFGTEPFTGSGTLNVASWTASIN

TreGlu4, Trichoderma reesei (SEQ ID NO:12):
HGHINDIVINGVWYQAYDPTTFPYESNPPIVVGWTAADLDNGFVSPDAYQNPDIICH
KNATNAKGHASVKAGDTILFQWVPVPWPHPGPIVDYLANCNGDCETVDKTTLEFF
KIDGVGLLSGGDPGTWASDVLISNNNTWVVKIPDNLAPGNYVLRHEIIALHSAGQA
NGAQNYPQCFNIAVSGSGSLQPSGVLGTDLYHATDPGVLINIYTSPLNYIIPGPTVVS
GLPTSVAQGSSAATATASATVPGGGSGPTSRTTTTARTTQASSRPSSTPPATTSAPAG
GPTQTLYGQCGGSGYSGPTRCAPPATCSTNPYYAQCLN

TreGlu6, Trichoderma reesei (SEQ ID NO:13):
AFSWKNVKLGGGGGFVPGIIFHPKTKGVAYARTDIGGLYRLNADDSWTAVTDGIA
DNAGWHNWGIDAVALDPQDDQKVYAAVGMYTNSWDPSNGAIIRSSDRGATWSFT
NLPFKVGGNMPGRGAGERLAVDPANSNIIYFGARSGNGLWKSTDGGVTFSKVSSFT
ATGTYIPDPSDSNGYNSDKQGLMWVTFDSTSSTTGGATSRIFVGTADNITASVYVST
NAGSTWSAVPGQPGKYFPHKAKLQPAEKALYLTYSWWPDAQLFRSTDSGTTWSPI
WAWASYPTETYYYSISTPKAPWIKNNFIDVTSESPSDGLIKRLGWMIESLEIDPTDSN
HWLYGTGMTIFGGHDLTNWDTRHNVSIQSLADGIEEFSVQDLASAPGGSELLAAVG
DDNGFTFASRNDLGTSPQTVWATPTWATSTSVDYAGNSVKSVVRVGNTAGTQQV
AISSDGGATWSIDYAADTSMNGGTVAYSADGDTILWSTASSGVQRSQFQGSFASVS
SLPAGAVIASDKKTNSVFYAGSGSTFYVSKDTGSSFTRGPKLGSAGTIRDIAAHPTT
AGTLYVSTDVGIFRSTDSGTTFGQVSTALTNTYQIALGVGSGSNWNLYAFGTGPSG
ARLYASGDSGASWTDIQGSQGFGSIDSTKVAGSGSTAGQVYVGTNGRGVFYAQGT
VGGGTGGTSSSTKQSSSSTSSASSSTTLRSSVVSTTRASTVTSSRTSSAAGPTGSGVA
GHYAQCGGIGWTGPTQCVAPYVCQKQNDYYYQCV

TreGlu7, Trichoderma reesei (SEQ ID NO:14):
HGQVQNFTINGQYNQGFILDYYYQKQNTGHFPNVAGWYAEDLDLGFISPDQYTTP
DIVCHKNAAPGAISATAAAGSNIVFQWGPGVWPHPYGPIVTYVVECSGSCTTVNKN
NLRWVKIQEAGINYNTQVWAQQDLINQGNKWTVKIPSSLRPGNYVFRHELLAAHG
ASSANGMQNYPQCVNIAVTGSGTKALPAGTPATQLYKPTDPGILFNPYTTITSYTIP
GPALWQG

TreGlu8, Trichoderma reesei (SEQ ID NO:15):
GKIKYLGVAIPGIDFGCDIDGSCPTDTSSVPLLSYKGGDGAGQMKHFAEDDGLNVF
RISATWQFVLNNTVDGKLDELNWGSYNKVVNACLETGAYCMIDMHNFARYNGGII
GQGGVSDDIFVDLWVQIAKYYEDNDKIIFGLMNEPHDLDIEIWAQTCQKVVTAIRK
AGATSQMILLPGTNFASVETYVSTGSAEALGKITNPDGSTDLLYFDVHKYLDINNSG
SHAECTTDNVDAFNDFADWLRQNKRQAIISETGASMEPSCMTAFCAQNKAISENSD
VYIGFVGWGAGSFDTSYILTLTPLGKPGNYTDNKLMNECILDQFTLDEKYRPTPTSI
STAAEETATATATSDGDAPSTTKPIFREETASPTPNAVTKPSPDTSDSSDDDKDSAAS
MSAQGLTGTVLFTVAALGYMLVAF

BsuGluC CBD, Bacillus subtilis (SEQ ID NO:16):
MKRSISIFITCLLITLLTMGGMIASPASAAGTKTPVAKNGQLSIKGTQLVNRDGKAV
QLKGISSHGLQWYGEYVNKDSLKWLRDDWGITVFRAAMYTADGGYIDNPSVKNK
VKEAVEAAKELGIYVIIDWHILNDGNPNQNKEKAKEFFKEMSSLYGNTPNVIYEIAN
EPNGDVNWKRDIKPYAEEVISVIRKNDPDNIIIVGTGTWSQDVNDAADDQLKDANV
MYALHFYAGTHGQFLRDKANYALSKGAPIFVTEWGTSDASGNGGVFLDQSREWL
KYLDSKTISWVNWNLSDKQESSSALKPGASKTGGWRLSDLSASGTFVRENILGTKD
STKDIPETPSKDKPTQENGISVQYRAGDGSMNSNQIRPQLQIKNNGNTTVDLKDVTA
RYWYKAKNKGQNFDCDYAQIGCGNVTHKFVTLHKPKQGADTYLELGFKNGTLAP
GASTGNIQLRLHNDDWSNYAQSGDYSFFKSNTFKTTKKITLYDQGKLIWGTEPN

BsuXyn3, Bacillus subtilis xylanase variant (SEQ ID NO:17):
ASTDYWQNWTFGGGIVNAVNGSGGNYSVNWSNTGNFVVGKGWTTGSPFRTINYN
AGVWAPNGNGYLTLYGWTRSPLIEYYVVDSWGTYRPTGTYKGTVKSDGGTYDIY
TTTRYNAPSIDGDDTTFTQYWSVRQSKRPTGSNATITFSNHVNAWKSHGMNLGSN
WAYQVMATEGYQSSGSSNVTVW

BsuXyn4, Bacillus subtilis xylanase variant (SEQ ID NO:18):
ASTDYWQNWTDGYGIVNAVNGSGGNYSVNWSNTGNFVVGKGWTTGSPFRTINYN
AGVWAPNGNGYLTLYGWTRSPLIEYYVVDSWGTYRPTGTYKGTVYSDGGWYDIY
TATRDNAPSIDGDFTTFTQYWSVRQSKRPTGSNATITFSNHVNAWRSHGMDLGSN
WAYQVMATEGYLSSGSSNVTVW

## Claims

1. A method of producing malted cereal comprising:
a) providing a cereal grain comprising one or more β-glucans and one or more arabinoxylans,
b) adding to said cereal an effective amount of a β-glucanase and an effective amount of a xylanase during a malting process; and
c) obtaining a malted cereal.

2. The method of any preceding claims, wherein said method comprises a steeping step including one or more wetting stages, where said β-glucanase and/or said xylanase are added one or more times during said one or more of wetting stages.

3. The method of claim 2, wherein:
(i) said β-glucanase and/or said xylanase are added at the beginning of the first wetting of the cereal; or
(ii) said β-glucanase and/or said xylanase are added during the first wetting of the cereal; or
(iii) said β-glucanase and/or said xylanase are added at the beginning of the second wetting of the cereal; or
(iv) said β-glucanase and/or said xylanase are added during the second wetting of the cereal; or
(v) said β-glucanase and/or said xylanase are added at the beginning of the third wetting of the cereal; or
(vi) said β-glucanase and/or said xylanase are added during the third wetting of the cereal; or
(vii) said β-glucanase and/or said xylanase are added at the beginning of all wetting stages of the cereal; or
(viii) said β-glucanase and/or said xylanase are added during all wetting stages of the cereal; or
(ix) said one or more wetting stages comprise spraying the cereal and wherein said β-glucanase and/or said xylanase are added one or more times, or constantly, during said spraying.

4. The method of any preceding claim, wherein the cereal reaches a moisture content of between about 40 to about 50 % W/W, wherein said moisture content is reached in less than 12 hours, and wherein said moisture content is reached with at least 10 % less water than the water used for a malted cereal prepared without said β-glucanase and/or said xylanase.

5. The method of any preceding claim, wherein:
(i) said cereal has 1-10 % W/W of β-glucans; or
(ii) said cereal has 1-10 % W/W of arabinoxylans; or
(iii) the amount of high molecular weight β-glucans in the cereal is decreased at least 50%; or
(iv) the amount of high molecular weight β-glucans in the cereal is decreased at least 80%; or
(v) the amount of high molecular weight arabinoxylans in the cereal is decreased at least 50%.; or
(vi) the cereal is selected from the group consisting of barley, wheat, rye, corn, oats, rice, millet, triticale, cassava, sorghum and a combination thereof; or
(vii) said β-glucanase is dosed at 0.01-100mg enzyme protein per kilogram of cereal; or
(viii) β-glucanase is dosed at 1-15 mg enzyme protein per kilogram of cereal; or
(ix) said xylanase is dosed at 0.01-100 mg enzyme protein per kilogram of cereal; or
(xi) said xylanase is dosed at 1-15 mg enzyme protein per kilogram of cereal.

6. The method of any preceding claim, wherein said β-glucanase is an endo-1,3(4)-β-glucanase.

7. The method of claim 6, wherein said endo-1,3(4)-β-glucanase enzyme comprises an amino acid sequence having at least 80% identity with any one selected from SEQ ID NO:7, SEQ ID NO:8, SEQ ID NO:9, SEQ ID NO:10, and SEQ ID NO: 11, SEQ ID NO:12, SEQ ID NO:13, SEQ ID NO:14, SEQ ID NO:15, SEQ ID NO:16, or any functional fragment thereof

8. The method of claims any preceding claim, wherein said xylanase is an endo-1,4-β-xylanase.

9. The method of claim 8, wherein said endo-1,4-β-xylanase enzyme comprises an amino acid sequence having at least 80% identity with any one selected from SEQ ID NO:1, SEQ ID NO:2, SEQ ID NO:3, SEQ ID NO:4, SEQ ID NO:5, SEQ ID NO:6, SEQ ID NO:17, and SEQ ID NO:18, or any functional fragment thereof

10. The method of any preceding claim, wherein said β-glucanase and said xylanase have an amino acid sequence having at least 80% sequence identity with the respective SEQ ID, or any functional fragment thereof, being selected from the list consisting of SEQ ID NO:1 and SEQ ID NO:7; SEQ ID NO:2 and SEQ ID NO:7; SEQ ID NO:3 and SEQ ID NO:7; SEQ ID NO:4 and SEQ ID NO:7; SEQ ID NO:5 and SEQ ID NO:7; SEQ ID NO:6 and SEQ ID NO:7; SEQ ID NO:17 and SEQ ID NO:7; SEQ ID NO:18 and SEQ ID NO:7; SEQ ID NO:1 and SEQ ID NO:8; SEQ ID NO:2 and SEQ ID NO:8; SEQ ID NO:3 and SEQ ID NO:8; SEQ ID NO:4 and SEQ ID NO:8; SEQ ID NO:5 and SEQ ID NO:8; SEQ ID NO:6 and SEQ ID NO:8; SEQ ID NO: 17 and SEQ ID NO:8; SEQ ID NO:18 and SEQ ID NO:8; SEQ ID NO:1 and SEQ ID NO:9; SEQ ID NO:2 and SEQ ID NO:9; SEQ ID NO:3 and SEQ ID NO:9; SEQ ID NO:4 and SEQ ID NO:9; SEQ ID NO:5 and SEQ ID NO:9; SEQ ID NO:6 and SEQ ID NO:9; SEQ ID NO:17 and SEQ ID NO:9; SEQ ID NO:18 and SEQ ID NO:9; SEQ ID NO:1 and SEQ ID NO: 10; SEQ ID NO:2 and SEQ ID NO: 10; SEQ ID NO:3 and SEQ ID NO:10; SEQ ID NO:4 and SEQ ID NO:10; SEQ ID NO:5 and SEQ ID NO:10; SEQ ID NO:6 and SEQ ID NO:10; SEQ ID NO:17 and SEQ ID NO:10; SEQ ID NO:18 and SEQ ID NO:10; SEQ ID NO:1 and SEQ ID NO:11; SEQ ID NO:2 and SEQ ID NO:11; SEQ ID NO:3 and SEQ ID NO:11; SEQ ID NO:4 and SEQ ID NO:11; SEQ ID NO:5 and SEQ ID NO:11; SEQ ID NO:6 and SEQ ID NO:11; SEQ ID NO:17 and SEQ ID NO:11; SEQ ID NO:18 and SEQ ID NO:11; SEQ ID NO:1 and SEQ ID NO:12; SEQ ID NO:2 and SEQ ID NO:12; SEQ ID NO:3 and SEQ ID NO:12; SEQ ID NO:4 and SEQ ID NO:12; SEQ ID NO:5 and SEQ ID NO:12; SEQ ID NO:6 and SEQ ID NO:12; SEQ ID NO: 17 and SEQ ID NO:12; SEQ ID NO:18 and SEQ ID NO:12; SEQ ID NO:1 and SEQ ID NO:13; SEQ ID NO:2 and SEQ ID NO:13; SEQ ID NO:3 and SEQ ID NO:13; SEQ ID NO:4 and SEQ ID NO:13; SEQ ID NO:5 and SEQ ID NO:13; SEQ ID NO:6 and SEQ ID NO:13; SEQ ID NO: 17 and SEQ ID NO: 13; SEQ ID NO:18 and SEQ ID NO: 13; SEQ ID NO:1 and
SEQ ID NO:14; SEQ ID NO:2 and SEQ ID NO:14; SEQ ID NO:3 and SEQ ID NO:14; SEQ ID NO:4 and SEQ ID NO:14; SEQ ID NO:5 and SEQ ID NO:14; SEQ ID NO:6 and SEQ ID NO:14; SEQ ID NO:17 and SEQ ID NO:14; SEQ ID NO:18 and SEQ ID NO:14; SEQ ID NO:1 and SEQ ID NO:15; SEQ ID NO:2 and SEQ ID NO:15; SEQ ID NO:3 and SEQ ID NO: 15; SEQ ID NO:4 and SEQ ID NO: 15; SEQ ID NO:5 and SEQ ID NO: 15; SEQ ID NO:6 and SEQ ID NO: 15; SEQ ID NO: 17 and SEQ ID NO: 15; SEQ ID NO:18 and SEQ ID NO:15; SEQ ID NO:1 and SEQ ID NO:16; SEQ ID NO:2 and SEQ ID NO:16; SEQ ID NO:3 and SEQ ID NO:16; SEQ ID NO:4 and SEQ ID NO:16; SEQ ID NO:5 and SEQ ID NO:16; SEQ ID NO:6 and SEQ ID NO:16; SEQ ID NO:17 and SEQ ID NO:16; and SEQ ID NO:18 and SEQ ID NO:16.

11. The method of any preceding claim, wherein said β-glucanase and said xylanase have an amino acid sequence having at least 80% sequence identity with the respective SEQ ID, or any functional fragment thereof, being selected from the list consisting of SEQ ID NO:1 and SEQ ID NO:7; SEQ ID NO:2 and SEQ ID NO:8; and SEQ ID NO:2 and SEQ ID NO:8.

12. The method of any preceding claim, wherein:
(i) said xylanase and said beta-glucanase are combined at a ratio of 1:1 per weight; or
(ii) said beta-glucanase has a beta-glucanase activity of at least 9000 U/g, and said xylanase has a xylanase activity of at least 13000U/g; or
(iii) said β-glucanase and said xylanase comprises commercially available enzymes having xylanase activity and/or beta-glucanase activity selected from the group consisting of UltraFlo L (available from Novozymes -beta glucanase with cellulase, xylanase side activities), UltraFlo XL (available from Novozymes -beta glucanase with xylanase and alpha amylase side activities), UltraFlo Max (available from Novozymes - beta glucanase and xylanase), Finizyme 250 L(available from Novozymes - beta glucanase with cellulase, xylanase side activities) and Filtrase series(available from DSM- betaglucanase and xylanases); or
(iv) a total steeping time is at least 10 % less than the time of a malted cereal prepared without said β-glucanase and/or said xylanase; or
(v) the total steeping time does not exceed 25 hours; or
(vi) the total steeping time does not exceed 12 hours; or
(vii) the total amount of water used during the steeping process is at least 10 % less than the amount of water used for a malted cereal prepared without said β-glucanase and/or said xylanase; or
(viii) the amount of high molecular weight β-glucans in the malted cereal is at least 10% less than the amount of high molecular weight β-glucans in a malted cereal prepared without said β-glucanase; or
(ix) the amount of high molecular weight arabinoxylans is at least 10 % less than the amount of arabinoxylans in a malted cereal prepared without said xylanase; or
(x) the amount of high molecular weight β-glucans and/or arabinoxylans is at least 50% less than the amount of β-glucans and/or arabinoxylans in a malted cereal prepared without said β-glucanase and/or said xylanase; or
(xi) the amount of high molecular weight β-glucans in said malted cereal is 50 mg/l or less, and the amount of arabinoxylans in said malted cereal is at 2000 mg/l or less; or
(xii) an extract prepared from said malted cereal has lower viscosity that an extract prepared with a malted cereal prepared without said β-glucanase and/or said xylanase; or
(xiii) the malted cereal has increased enzymatic activity.

13. The method of claim 12 (xiii), wherein:
(a) said enzymatic activity is increased by a factor of at least about 2 as compared to a malted cereal prepared without said β-glucanase and/or said xylanase; or
(b) said enzymatic activity is selected from the group consisting of β-glucanase activity, xylanase activity, amylase activity, protease activity, naturally occurring enzyme activity in the cereal and combinations thereof; or
(c) said enzymatic activity is β-glucanase and/or xylanase activity.

14. The method according to any preceding claim, further comprising adding one or more enzymes selected from the group of amylase, a protease, naturally occurring enzymes in the cereal and combinations thereof.

15. The method according to any preceding claim, said method comprises a steeping step including one or more wetting stages, where said β-glucanase and/or said xylanase are added one or more times during said one or more of wetting stages, and wherein the amount of high molecular weight β-glucans in said malted cereal is 50 mg/l or less, and the amount of arabinoxylans in said malted cereal is at 2000 mg/l or less.


SEQUENCE LISTING

<110>  DuPont Nutrition Biosciences ApS

<120>  Enzymes

<130>  18733PCT00

<160>  18

<170>  PatentIn version 3.5

<210>  1
<211>  302
<212>  PRT
<213>  Aspergillus tubigensis

<400>  1

Gln Ala Ser Val Ser Ile Asp Thr Lys Phe Lys Ala His Gly Lys Lys
1               5                   10                  15

Tyr Leu Gly Asn Ile Gly Asp Gln Tyr Thr Leu Thr Lys Asn Ser Lys
            20                  25                  30

Thr Pro Ala Ile Ile Lys Ala Asp Phe Gly Ala Leu Thr Pro Glu Asn
        35                  40                  45

Ser Met Lys Trp Asp Ala Thr Glu Pro Ser Arg Gly Gln Phe Ser Phe
    50                  55                  60

Ser Gly Ser Asp Tyr Leu Val Asn Phe Ala Gln Ser Asn Asn Lys Leu
65                  70                  75                  80

Ile Arg Gly His Thr Leu Val Trp His Ser Gln Leu Pro Ser Trp Val
                85                  90                  95

Gln Ala Ile Thr Asp Lys Asn Thr Leu Ile Glu Val Met Lys Asn His
            100                 105                 110

Ile Thr Thr Val Met Gln His Tyr Lys Gly Lys Ile Tyr Ala Trp Asp
        115                 120                 125

Val Val Asn Glu Ile Phe Asn Glu Asp Gly Ser Leu Arg Asp Ser Val
    130                 135                 140

Phe Tyr Gln Val Ile Gly Glu Asp Tyr Val Arg Ile Ala Phe Glu Thr
145                 150                 155                 160

Ala Arg Ala Ala Asp Pro Asn Ala Lys Leu Tyr Ile Asn Asp Tyr Asn
                165                 170                 175

Leu Asp Ser Ala Ser Tyr Pro Lys Leu Thr Gly Met Val Ser His Val
            180                 185                 190

Lys Lys Trp Ile Glu Ala Gly Ile Pro Ile Asp Gly Ile Gly Ser Gln
        195                 200                 205

Thr His Leu Ser Ala Gly Gly Gly Ala Gly Ile Ser Gly Ala Leu Asn
    210                 215                 220

Ala Leu Ala Gly Ala Gly Thr Lys Glu Ile Ala Val Thr Glu Leu Asp
225                 230                 235                 240

Ile Ala Gly Ala Ser Ser Thr Asp Tyr Val Glu Val Val Glu Ala Cys
                245                 250                 255

Leu Asp Gln Pro Lys Cys Ile Gly Ile Thr Val Trp Gly Val Ala Asp
            260                 265                 270

Pro Asp Ser Trp Arg Ser Ser Ser Thr Pro Leu Leu Phe Asp Ser Asn
        275                 280                 285

Tyr Asn Pro Lys Pro Ala Tyr Thr Ala Ile Ala Asn Ala Leu
    290                 295                 300

<210>  2
<211>  386
<212>  PRT
<213>  Geosmithia emersonii

<400>  2

Ala Gly Leu Asn Thr Ala Ala Lys Ala Ile Gly Leu Lys Tyr Phe Gly
1               5                   10                  15

Thr Ala Thr Asp Asn Pro Glu Leu Ser Asp Thr Ala Tyr Glu Thr Gln
            20                  25                  30

Leu Asn Asn Thr Gln Asp Phe Gly Gln Leu Thr Pro Ala Asn Ser Met
        35                  40                  45

Lys Trp Asp Ala Thr Glu Pro Glu Gln Asn Val Phe Thr Phe Ser Ala
    50                  55                  60

Gly Asp Gln Ile Ala Asn Leu Ala Lys Ala Asn Gly Gln Met Leu Arg
65                  70                  75                  80

Cys His Asn Leu Val Trp Tyr Asn Gln Leu Pro Ser Trp Val Thr Ser
                85                  90                  95

Gly Ser Trp Thr Asn Glu Thr Leu Leu Ala Ala Met Lys Asn His Ile
            100                 105                 110

Thr Asn Val Val Thr His Tyr Lys Gly Gln Cys Tyr Ala Trp Asp Val
        115                 120                 125

Val Asn Glu Ala Leu Asn Asp Asp Gly Thr Tyr Arg Ser Asn Val Phe
    130                 135                 140

Tyr Gln Tyr Ile Gly Glu Ala Tyr Ile Pro Ile Ala Phe Ala Thr Ala
145                 150                 155                 160

Ala Ala Ala Asp Pro Asn Ala Lys Leu Tyr Tyr Asn Asp Tyr Asn Ile
                165                 170                 175

Glu Tyr Pro Gly Ala Lys Ala Thr Ala Ala Gln Asn Leu Val Lys Leu
            180                 185                 190

Val Gln Ser Tyr Gly Ala Arg Ile Asp Gly Val Gly Leu Gln Ser His
        195                 200                 205

Phe Ile Val Gly Glu Thr Pro Ser Thr Ser Ser Gln Gln Gln Asn Met
    210                 215                 220

Ala Ala Phe Thr Ala Leu Gly Val Glu Val Ala Ile Thr Glu Leu Asp
225                 230                 235                 240

Ile Arg Met Gln Leu Pro Glu Thr Glu Ala Leu Leu Thr Gln Gln Ala
                245                 250                 255

Thr Asp Tyr Gln Ser Thr Val Gln Ala Cys Ala Asn Thr Lys Gly Cys
            260                 265                 270

Val Gly Ile Thr Val Trp Asp Trp Thr Asp Lys Tyr Ser Trp Val Pro
        275                 280                 285

Ser Thr Phe Ser Gly Tyr Gly Asp Ala Cys Pro Trp Asp Ala Asn Tyr
    290                 295                 300

Gln Lys Lys Pro Ala Tyr Glu Gly Ile Leu Thr Gly Leu Gly Gln Thr
305                 310                 315                 320

Val Thr Ser Thr Thr Tyr Ile Ile Ser Pro Thr Thr Ser Val Gly Thr
                325                 330                 335

Gly Thr Thr Thr Ser Ser Gly Gly Ser Gly Gly Thr Thr Gly Val Ala
            340                 345                 350

Gln His Trp Glu Gln Cys Gly Gly Leu Gly Trp Thr Gly Pro Thr Val
        355                 360                 365

Cys Ala Ser Gly Tyr Thr Cys Thr Val Ile Asn Glu Tyr Tyr Ser Gln
    370                 375                 380

Cys Leu
385

<210> 3
<211> 308
<212> PRT
<213> Aspergillus tubigensis

<400> 3

Glu Pro Ile Glu Pro Arg Gln Ala Ser Val Ser Ile Asp Thr Lys Phe
1               5                   10                  15

Lys Ala His Gly Lys Lys Tyr Leu Gly Asn Ile Gly Asp Gln Tyr Thr
            20                  25                  30

Leu Thr Lys Asn Ser Lys Thr Pro Ala Ile Ile Lys Ala Asp Phe Gly
        35                  40                  45

Ala Leu Thr Pro Glu Asn Ser Met Lys Trp Asp Ala Thr Glu Pro Ser
    50                  55                  60

Arg Gly Gln Phe Ser Phe Ser Gly Ser Asp Tyr Leu Val Asn Phe Ala
65                  70                  75                  80

Gln Ser Asn Asn Lys Leu Ile Arg Gly His Thr Leu Val Trp His Ser
                85                  90                  95

Gln Leu Pro Ser Trp Val Gln Ser Ile Thr Asp Lys Asn Thr Leu Ile
            100                 105                 110

Glu Val Met Lys Asn His Ile Thr Thr Val Met Gln His Tyr Lys Gly
        115                 120                 125

Lys Ile Tyr Ala Trp Asp Val Val Asn Glu Ile Phe Asn Glu Asp Gly
    130                 135                 140

Ser Leu Arg Asp Ser Val Phe Tyr Lys Val Ile Gly Glu Asp Tyr Val
145                 150                 155                 160

Arg Ile Ala Phe Glu Thr Ala Arg Ala Ala Asp Pro Asn Ala Lys Leu
                165                 170                 175

Tyr Ile Asn Asp Tyr Asn Leu Asp Ser Ala Ser Tyr Pro Lys Leu Thr
            180                 185                 190

Gly Met Val Ser His Val Lys Lys Trp Ile Ala Ala Gly Ile Pro Ile
        195                 200                 205

Asp Gly Ile Gly Ser Gln Thr His Leu Ser Ala Gly Gly Gly Ala Gly
    210                 215                 220

Ile Ser Gly Ala Leu Asn Ala Leu Ala Gly Ala Gly Thr Lys Glu Ile
225                 230                 235                 240

Ala Val Thr Glu Leu Asp Ile Ala Gly Ala Ser Ser Thr Asp Tyr Val
                245                 250                 255

Glu Val Val Glu Ala Cys Leu Asn Gln Pro Lys Cys Ile Gly Ile Thr
            260                 265                 270

Val Trp Gly Val Ala Asp Pro Asp Ser Trp Arg Ser Ser Ser Thr Pro
        275                 280                 285

Leu Leu Phe Asp Ser Asn Tyr Asn Pro Lys Pro Ala Tyr Thr Ala Ile
    290                 295                 300

Ala Asn Ala Leu
305

<210>  4
<211>  406
<212>  PRT
<213>  Aspergillus aculeatus

<400>  4

Met Val Gly Leu Leu Ser Ile Thr Ala Ala Leu Ala Ala Thr Val Leu
1               5                   10                  15

Pro Asn Ile Val Ser Ala Val Gly Leu Asp Gln Ala Ala Val Ala Lys
            20                  25                  30

Gly Leu Gln Tyr Phe Gly Thr Ala Thr Asp Asn Pro Glu Leu Thr Asp
        35                  40                  45

Ile Pro Tyr Val Thr Gln Leu Asn Asn Thr Ala Asp Phe Gly Gln Ile
    50                  55                  60

Thr Pro Gly Asn Ser Met Lys Trp Asp Ala Thr Glu Pro Ser Gln Gly
65                  70                  75                  80

Thr Phe Thr Phe Thr Lys Gly Asp Val Ile Ala Asp Leu Ala Glu Gly
                85                  90                  95

Asn Gly Gln Tyr Leu Arg Cys His Thr Leu Val Trp Tyr Asn Gln Leu
            100                 105                 110

Pro Ser Trp Val Thr Ser Gly Thr Trp Thr Asn Ala Thr Leu Thr Ala
        115                 120                 125

Ala Leu Lys Asn His Ile Thr Asn Val Val Ser His Tyr Lys Gly Lys
    130                 135                 140

Cys Leu His Trp Asp Val Val Asn Glu Ala Leu Asn Asp Asp Gly Thr
145                 150                 155                 160

Tyr Arg Thr Asn Ile Phe Tyr Thr Thr Ile Gly Glu Ala Tyr Ile Pro
                165                 170                 175

Ile Ala Phe Ala Ala Ala Ala Ala Ala Asp Pro Asp Ala Lys Leu Phe
            180                 185                 190

Tyr Asn Asp Tyr Asn Leu Glu Tyr Gly Gly Ala Lys Ala Ala Ser Ala
        195                 200                 205

Arg Ala Ile Val Gln Leu Val Lys Asn Ala Gly Ala Lys Ile Asp Gly
    210                 215                 220

Val Gly Leu Gln Ala His Phe Ser Val Gly Thr Val Pro Ser Thr Ser
225                 230                 235                 240

Ser Leu Val Ser Val Leu Gln Ser Phe Thr Ala Leu Gly Val Glu Val
                245                 250                 255

Ala Tyr Thr Glu Ala Asp Val Arg Ile Leu Leu Pro Thr Thr Ala Thr
            260                 265                 270

Thr Leu Ala Gln Gln Ser Ser Asp Phe Gln Ala Leu Val Gln Ser Cys
        275                 280                 285

Val Gln Thr Thr Gly Cys Val Gly Phe Thr Ile Trp Asp Trp Thr Asp
    290                 295                 300

Lys Tyr Ser Trp Val Pro Ser Thr Phe Ser Gly Tyr Gly Ala Ala Leu
305                 310                 315                 320

Pro Trp Asp Glu Asn Leu Val Lys Lys Pro Ala Tyr Asn Gly Leu Leu
                325                 330                 335

Ala Gly Met Gly Val Thr Val Thr Thr Thr Thr Thr Thr Thr Thr Ala
        340                 345                 350

Thr Ala Thr Gly Lys Thr Thr Thr Thr Thr Thr Gly Ala Thr Ser Thr
    355                 360                 365

Gly Thr Thr Ala Ala His Trp Gly Gln Cys Gly Gly Leu Asn Trp Ser
370                 375                 380

Gly Pro Thr Ala Cys Ala Thr Gly Tyr Thr Cys Thr Tyr Val Asn Asp
385                 390                 395                 400

Tyr Tyr Ser Gln Cys Leu
                405

<210>  5
<211>  347
<212>  PRT
<213>  Trichoderma reesei

<400>  5

Met Lys Ala Asn Val Ile Leu Cys Leu Leu Ala Pro Leu Val Ala Ala
1               5                   10                  15

Leu Pro Thr Glu Thr Ile His Leu Asp Pro Glu Leu Ala Ala Leu Arg
            20                  25                  30

Ala Asn Leu Thr Glu Arg Thr Ala Asp Leu Trp Asp Arg Gln Ala Ser
        35                  40                  45

Gln Ser Ile Asp Gln Leu Ile Lys Arg Lys Gly Lys Leu Tyr Phe Gly
    50                  55                  60

Thr Ala Thr Asp Arg Gly Leu Leu Gln Arg Glu Lys Asn Ala Ala Ile
65                  70                  75                  80

Ile Gln Ala Asp Leu Gly Gln Val Thr Pro Glu Asn Ser Met Lys Trp
                85                  90                  95

Gln Ser Leu Glu Asn Asn Gln Gly Gln Leu Asn Trp Gly Asp Ala Asp
            100                 105                 110

Tyr Leu Val Asn Phe Ala Gln Gln Asn Gly Lys Ser Ile Arg Gly His
        115                 120                 125

Thr Leu Ile Trp His Ser Gln Leu Pro Ala Trp Val Asn Asn Ile Asn
    130                 135                 140

Asn Ala Asp Thr Leu Arg Gln Val Ile Arg Thr His Val Ser Thr Val
145                 150                 155                 160

Val Gly Arg Tyr Lys Gly Lys Ile Arg Ala Trp Asp Val Val Asn Glu
                165                 170                 175

Ile Phe Asn Glu Asp Gly Thr Leu Arg Ser Ser Val Phe Ser Arg Leu
            180                 185                 190

Leu Gly Glu Glu Phe Val Ser Ile Ala Phe Arg Ala Ala Arg Asp Ala
        195                 200                 205

Asp Pro Ser Ala Arg Leu Tyr Ile Asn Asp Tyr Asn Leu Asp Arg Ala
    210                 215                 220

Asn Tyr Gly Lys Val Asn Gly Leu Lys Thr Tyr Val Ser Lys Trp Ile
225                 230                 235                 240

Ser Gln Gly Val Pro Ile Asp Gly Ile Gly Ser Gln Ser His Leu Ser
                245                 250                 255

Gly Gly Gly Gly Ser Gly Thr Leu Gly Ala Leu Gln Gln Leu Ala Thr
            260                 265                 270

Val Pro Val Thr Glu Leu Ala Ile Thr Glu Leu Asp Ile Gln Gly Ala
        275                 280                 285

Pro Thr Thr Asp Tyr Thr Gln Val Val Gln Ala Cys Leu Ser Val Ser
    290                 295                 300

Lys Cys Val Gly Ile Thr Val Trp Gly Ile Ser Asp Lys Asp Ser Trp
305                 310                 315                 320

Arg Ala Ser Thr Asn Pro Leu Leu Phe Asp Ala Asn Phe Asn Pro Lys
                325                 330                 335

Pro Ala Tyr Asn Ser Ile Val Gly Ile Leu Gln
            340                 345

<210>  6
<211>  191
<212>  PRT
<213>  Trichoderma reesei

<400>  6

Gln Cys Ile Gln Pro Gly Thr Gly Tyr Asn Asn Gly Tyr Phe Tyr Ser
1               5                   10                  15

Tyr Trp Asn Asp Gly His Gly Gly Val Thr Tyr Cys Asn Gly Pro Gly
            20                  25                  30

Gly Gln Phe Ser Val Asn Trp Ser Asn Ser Gly Asn Phe Val Gly Gly
        35                  40                  45

Lys Gly Trp Gln Pro Gly Thr Lys Asn Arg Val Ile Asn Phe Ser Gly
    50                  55                  60

Ser Tyr Asn Pro Asn Gly Asn Ser Tyr Leu Ser Val Tyr Gly Trp Ser
65                  70                  75                  80

Arg Asn Pro Leu Ile Glu Tyr Tyr Ile Val Glu Asn Phe Gly Thr Tyr
                85                  90                  95

Asn Pro Ser Thr Gly Ala Thr Lys Leu Gly Glu Val Thr Ser Asp Gly
            100                 105                 110

Ser Val Tyr Asp Ile Tyr Arg Thr Gln Arg Val Asn Gln Pro Ser Ile
        115                 120                 125

Ile Gly Thr Ala Thr Phe Tyr Gln Tyr Trp Ser Val Arg Arg Asn His
    130                 135                 140

Arg Ser Ser Gly Ser Val Asn Thr Ala Asn His Phe Asn Ala Trp Ala
145                 150                 155                 160

Gln Gln Gly Leu Thr Leu Gly Thr Met Asp Tyr Gln Ile Val Ala Val
                165                 170                 175

Glu Gly Tyr Phe Ser Ser Gly Ser Ala Ser Ile Thr Val Ser Asp
            180                 185                 190

<210>  7
<211>  214
<212>  PRT
<213>  Bacillus subtilis

<400>  7

Gln Thr Gly Gly Ser Phe Phe Asp Pro Phe Asn Gly Tyr Asn Ser Gly
1               5                   10                  15

Phe Trp Gln Lys Ala Asp Gly Tyr Ser Asn Gly Asn Met Phe Asn Cys
            20                  25                  30

Thr Trp Arg Ala Asn Asn Val Ser Met Thr Ser Leu Gly Glu Met Arg
        35                  40                  45

Leu Ala Leu Thr Ser Pro Ala Tyr Asn Lys Phe Asp Cys Gly Glu Asn
    50                  55                  60

Arg Ser Val Gln Thr Tyr Gly Tyr Gly Leu Tyr Glu Val Arg Met Lys
65                  70                  75                  80

Pro Ala Lys Asn Thr Gly Ile Val Ser Ser Phe Phe Thr Tyr Thr Gly
                85                  90                  95

Pro Thr Asp Gly Thr Pro Trp Asp Glu Ile Asp Ile Glu Phe Leu Gly
            100                 105                 110

Lys Asp Thr Thr Lys Val Gln Phe Asn Tyr Tyr Thr Asn Gly Ala Gly
        115                 120                 125

Asn His Glu Lys Ile Val Asp Leu Gly Phe Asp Ala Ala Asn Ala Tyr
    130                 135                 140

His Thr Tyr Ala Phe Asp Trp Gln Pro Asn Ser Ile Lys Trp Tyr Val
145                 150                 155                 160

Asp Gly Gln Leu Lys His Thr Ala Thr Asn Gln Ile Pro Thr Thr Pro
                165                 170                 175

Gly Lys Ile Met Met Asn Leu Trp Asn Gly Thr Gly Val Asp Glu Trp
            180                 185                 190

Leu Gly Ser Tyr Asn Gly Val Asn Pro Leu Tyr Ala His Tyr Asp Trp
        195                 200                 205

Val Arg Tyr Thr Lys Lys
    210

<210>  8
<211>  316
<212>  PRT
<213>  Geosmithia emersonii

<400>  8

Ala Pro Val Lys Glu Lys Gly Ile Lys Lys Arg Ala Ser Pro Phe Gln
1               5                   10                  15

Trp Phe Gly Ser Asn Glu Ser Gly Ala Glu Phe Gly Asn Asn Asn Ile
            20                  25                  30

Pro Gly Val Glu Gly Thr Asp Tyr Thr Phe Pro Asn Thr Ser Ala Ile
        35                  40                  45

Gln Ile Leu Ile Asp Gln Gly Met Asn Ile Phe Arg Val Pro Phe Leu
    50                  55                  60

Met Glu Arg Met Val Pro Asn Gln Met Thr Gly Pro Val Asp Ser Ala
65                  70                  75                  80

Tyr Phe Gln Gly Tyr Ser Gln Val Ile Asn Tyr Ile Thr Ser His Gly
                85                  90                  95

Ala Ser Ala Val Ile Asp Pro His Asn Phe Gly Arg Tyr Tyr Asn Asn
            100                 105                 110

Ile Ile Ser Ser Pro Ser Asp Phe Gln Thr Phe Trp His Thr Ile Ala
        115                 120                 125

Ser Asn Phe Ala Asp Asn Asp Asn Val Ile Phe Asp Thr Asn Asn Glu
    130                 135                 140

Tyr His Asp Met Asp Glu Ser Leu Val Val Gln Leu Asn Gln Ala Ala
145                 150                 155                 160

Ile Asp Gly Ile Arg Ala Ala Gly Ala Thr Ser Gln Tyr Ile Phe Val
                165                 170                 175

Glu Gly Asn Ser Trp Thr Gly Ala Trp Thr Trp Thr Gln Val Asn Asp
            180                 185                 190

Ala Met Ala Asn Leu Thr Asp Pro Gln Asn Lys Ile Val Tyr Glu Met
        195                 200                 205

His Gln Tyr Leu Asp Ser Asp Gly Ser Gly Thr Ser Asp Gln Cys Val
    210                 215                 220

Asn Ser Thr Ile Gly Gln Asp Arg Val Glu Ser Ala Thr Ala Trp Leu
225                 230                 235                 240

Lys Gln Asn Gly Lys Lys Ala Ile Leu Gly Glu Tyr Ala Gly Gly Ala
                245                 250                 255

Asn Ser Val Cys Glu Thr Ala Val Thr Gly Met Leu Asp Tyr Leu Ala
            260                 265                 270

Asn Asn Thr Asp Val Trp Thr Gly Ala Ile Trp Trp Ala Ala Gly Pro
        275                 280                 285

Trp Trp Gly Asp Tyr Ile Phe Ser Met Glu Pro Pro Ser Gly Ile Ala

290                 295                 300

Tyr Glu Gln Val Leu Pro Leu Leu Gln Pro Tyr Leu
305                 310                 315

<210>  9
<211>  441
<212>  PRT
<213>  Bacillus subtilis

<400>  9

Asp Asp Tyr Ser Val Val Glu Glu His Gly Gln Leu Ser Ile Ser Asn
1               5                   10                  15

Gly Glu Leu Val Asn Glu Arg Gly Glu Gln Val Gln Leu Lys Gly Met
            20                  25                  30

Ser Ser His Gly Leu Gln Trp Tyr Gly Gln Phe Val Asn Tyr Glu Ser
        35                  40                  45

Met Lys Trp Leu Arg Asp Asp Trp Gly Ile Thr Val Phe Arg Ala Ala
    50                  55                  60

Met Tyr Thr Ser Ser Gly Gly Tyr Ile Asp Asp Pro Ser Val Lys Glu
65                  70                  75                  80

Lys Val Lys Glu Thr Val Glu Ala Ala Ile Asp Leu Gly Ile Tyr Val
                85                  90                  95

Ile Ile Asp Trp His Ile Leu Ser Asp Asn Asp Pro Asn Ile Tyr Lys
            100                 105                 110

Glu Glu Ala Lys Asp Phe Phe Asp Glu Met Ser Glu Leu Tyr Gly Asp
        115                 120                 125

Tyr Pro Asn Val Ile Tyr Glu Ile Ala Asn Glu Pro Asn Gly Ser Asp
    130                 135                 140

Val Thr Trp Asp Asn Gln Ile Lys Pro Tyr Ala Glu Glu Val Ile Pro
145                 150                 155                 160

Val Ile Arg Asp Asn Asp Pro Asn Asn Ile Val Ile Val Gly Thr Gly
                165                 170                 175

Thr Trp Ser Gln Asp Val His His Ala Ala Asp Asn Gln Leu Ala Asp
            180                 185                 190

Pro Asn Val Met Tyr Ala Phe His Phe Tyr Ala Gly Thr His Gly Gln

195                 200                 205

Asn Leu Arg Asp Gln Val Asp Tyr Ala Leu Asp Gln Gly Ala Ala Ile
    210                 215                 220

Phe Val Ser Glu Trp Gly Thr Ser Ala Ala Thr Gly Asp Gly Gly Val
225                 230                 235                 240

Phe Leu Asp Glu Ala Gln Val Trp Ile Asp Phe Met Asp Glu Arg Asn
                245                 250                 255

Leu Ser Trp Ala Asn Trp Ser Leu Thr His Lys Asp Glu Ser Ser Ala
            260                 265                 270

Ala Leu Met Pro Gly Ala Asn Pro Thr Gly Gly Trp Thr Glu Ala Glu
        275                 280                 285

Leu Ser Pro Ser Gly Thr Phe Val Arg Glu Lys Ile Arg Glu Ser Ala
    290                 295                 300

Ser Ile Pro Pro Ser Asp Pro Thr Pro Pro Ser Asp Pro Gly Glu Pro
305                 310                 315                 320

Asp Pro Gly Glu Pro Asp Pro Thr Pro Pro Ser Asp Pro Gly Glu Tyr
                325                 330                 335

Pro Ala Trp Asp Ser Asn Gln Ile Tyr Thr Asn Glu Ile Val Tyr His
            340                 345                 350

Asn Gly Gln Leu Trp Gln Ala Lys Trp Trp Thr Gln Asn Gln Glu Pro
        355                 360                 365

Gly Asp Pro Tyr Gly Pro Trp Glu Pro Leu Lys Ser Asp Pro Asp Ser
    370                 375                 380

Gly Glu Pro Asp Pro Thr Pro Pro Ser Asp Pro Gly Glu Tyr Pro Ala
385                 390                 395                 400

Trp Asp Ser Asn Gln Ile Tyr Thr Asn Glu Ile Val Tyr His Asn Gly
                405                 410                 415

Gln Leu Trp Gln Ala Lys Trp Trp Thr Gln Asn Gln Glu Pro Gly Asp
            420                 425                 430

Pro Tyr Gly Pro Trp Glu Pro Leu Asn
        435                 440

<210>  10
<211>  397
<212>  PRT
<213>  Trichoderma reesei

<400>  10

Gln Gln Thr Val Trp Gly Gln Cys Gly Gly Ile Gly Trp Ser Gly Pro
1               5                   10                  15

Thr Asn Cys Ala Pro Gly Ser Ala Cys Ser Thr Leu Asn Pro Tyr Tyr
            20                  25                  30

Ala Gln Cys Ile Pro Gly Ala Thr Thr Ile Thr Thr Ser Thr Arg Pro
        35                  40                  45

Pro Ser Gly Pro Thr Thr Thr Thr Arg Ala Thr Ser Thr Ser Ser Ser
    50                  55                  60

Thr Pro Pro Thr Ser Ser Gly Val Arg Phe Ala Gly Val Asn Ile Ala
65                  70                  75                  80

Gly Phe Asp Phe Gly Cys Thr Thr Asp Gly Thr Cys Val Thr Ser Lys
                85                  90                  95

Val Tyr Pro Pro Leu Lys Asn Phe Thr Gly Ser Asn Asn Tyr Pro Asp
            100                 105                 110

Gly Ile Gly Gln Met Gln His Phe Val Asn Asp Asp Gly Met Thr Ile
        115                 120                 125

Phe Arg Leu Pro Val Gly Trp Gln Tyr Leu Val Asn Asn Asn Leu Gly
    130                 135                 140

Gly Asn Leu Asp Ser Thr Ser Ile Ser Lys Tyr Asp Gln Leu Val Gln
145                 150                 155                 160

Gly Cys Leu Ser Leu Gly Ala Tyr Cys Ile Val Asp Ile His Asn Tyr
                165                 170                 175

Ala Arg Trp Asn Gly Gly Ile Ile Gly Gln Gly Gly Pro Thr Asn Ala
            180                 185                 190

Gln Phe Thr Ser Leu Trp Ser Gln Leu Ala Ser Lys Tyr Ala Ser Gln
        195                 200                 205

Ser Arg Val Trp Phe Gly Ile Met Asn Glu Pro His Asp Val Asn Ile
    210                 215                 220

Asn Thr Trp Ala Ala Thr Val Gln Glu Val Val Thr Ala Ile Arg Asn
225                 230                 235                 240

Ala Gly Ala Thr Ser Gln Phe Ile Ser Leu Pro Gly Asn Asp Trp Gln
                245                 250                 255

Ser Ala Gly Ala Phe Ile Ser Asp Gly Ser Ala Ala Ala Leu Ser Gln
            260                 265                 270

Val Thr Asn Pro Asp Gly Ser Thr Thr Asn Leu Ile Phe Asp Val His
        275                 280                 285

Lys Tyr Leu Asp Ser Asp Asn Ser Gly Thr His Ala Glu Cys Thr Thr
    290                 295                 300

Asn Asn Ile Asp Gly Ala Phe Ser Pro Leu Ala Thr Trp Leu Arg Gln
305                 310                 315                 320

Asn Asn Arg Gln Ala Ile Leu Thr Glu Thr Gly Gly Gly Asn Val Gln
                325                 330                 335

Ser Cys Ile Gln Asp Met Cys Gln Gln Ile Gln Tyr Leu Asn Gln Asn
            340                 345                 350

Ser Asp Val Tyr Leu Gly Tyr Val Gly Trp Gly Ala Gly Ser Phe Asp
        355                 360                 365

Ser Thr Tyr Val Leu Thr Glu Thr Pro Thr Gly Ser Gly Asn Ser Trp
    370                 375                 380

Thr Asp Thr Ser Leu Val Ser Ser Cys Leu Ala Arg Lys
385                 390                 395

<210>  11
<211>  218
<212>  PRT
<213>  Trichoderma reesei

<400>  11

Gln Thr Ser Cys Asp Gln Trp Ala Thr Phe Thr Gly Asn Gly Tyr Thr
1               5                   10                  15

Val Ser Asn Asn Leu Trp Gly Ala Ser Ala Gly Ser Gly Phe Gly Cys
            20                  25                  30

Val Thr Ala Val Ser Leu Ser Gly Gly Ala Ser Trp His Ala Asp Trp
        35                  40                  45

Gln Trp Ser Gly Gly Gln Asn Asn Val Lys Ser Tyr Gln Asn Ser Gln
    50                  55                  60

Ile Ala Ile Pro Gln Lys Arg Thr Val Asn Ser Ile Ser Ser Met Pro
65                  70                  75                  80

Thr Thr Ala Ser Trp Ser Tyr Ser Gly Ser Asn Ile Arg Ala Asn Val
                85                  90                  95

Ala Tyr Asp Leu Phe Thr Ala Ala Asn Pro Asn His Val Thr Tyr Ser
            100                 105                 110

Gly Asp Tyr Glu Leu Met Ile Trp Leu Gly Lys Tyr Gly Asp Ile Gly
        115                 120                 125

Pro Ile Gly Ser Ser Gln Gly Thr Val Asn Val Gly Gly Gln Ser Trp
    130                 135                 140

Thr Leu Tyr Tyr Gly Tyr Asn Gly Ala Met Gln Val Tyr Ser Phe Val
145                 150                 155                 160

Ala Gln Thr Asn Thr Thr Asn Tyr Ser Gly Asp Val Lys Asn Phe Phe
                165                 170                 175

Asn Tyr Leu Arg Asp Asn Lys Gly Tyr Asn Ala Ala Gly Gln Tyr Val
            180                 185                 190

Leu Ser Tyr Gln Phe Gly Thr Glu Pro Phe Thr Gly Ser Gly Thr Leu
        195                 200                 205

Asn Val Ala Ser Trp Thr Ala Ser Ile Asn
    210                 215

<210>  12
<211>  322
<212>  PRT
<213>  Trichoderma reesei

<400>  12

His Gly His Ile Asn Asp Ile Val Ile Asn Gly Val Trp Tyr Gln Ala
1               5                   10                  15

Tyr Asp Pro Thr Thr Phe Pro Tyr Glu Ser Asn Pro Pro Ile Val Val
            20                  25                  30

Gly Trp Thr Ala Ala Asp Leu Asp Asn Gly Phe Val Ser Pro Asp Ala
        35                  40                  45

Tyr Gln Asn Pro Asp Ile Ile Cys His Lys Asn Ala Thr Asn Ala Lys
    50                  55                  60

Gly His Ala Ser Val Lys Ala Gly Asp Thr Ile Leu Phe Gln Trp Val
65                  70                  75                  80

Pro Val Pro Trp Pro His Pro Gly Pro Ile Val Asp Tyr Leu Ala Asn
                85                  90                  95

Cys Asn Gly Asp Cys Glu Thr Val Asp Lys Thr Thr Leu Glu Phe Phe
            100                 105                 110

Lys Ile Asp Gly Val Gly Leu Leu Ser Gly Gly Asp Pro Gly Thr Trp
        115                 120                 125

Ala Ser Asp Val Leu Ile Ser Asn Asn Asn Thr Trp Val Val Lys Ile
    130                 135                 140

Pro Asp Asn Leu Ala Pro Gly Asn Tyr Val Leu Arg His Glu Ile Ile
145                 150                 155                 160

Ala Leu His Ser Ala Gly Gln Ala Asn Gly Ala Gln Asn Tyr Pro Gln
                165                 170                 175

Cys Phe Asn Ile Ala Val Ser Gly Ser Gly Ser Leu Gln Pro Ser Gly
            180                 185                 190

Val Leu Gly Thr Asp Leu Tyr His Ala Thr Asp Pro Gly Val Leu Ile
        195                 200                 205

Asn Ile Tyr Thr Ser Pro Leu Asn Tyr Ile Ile Pro Gly Pro Thr Val
    210                 215                 220

Val Ser Gly Leu Pro Thr Ser Val Ala Gln Gly Ser Ser Ala Ala Thr
225                 230                 235                 240

Ala Thr Ala Ser Ala Thr Val Pro Gly Gly Gly Ser Gly Pro Thr Ser
                245                 250                 255

Arg Thr Thr Thr Thr Ala Arg Thr Thr Gln Ala Ser Ser Arg Pro Ser
            260                 265                 270

Ser Thr Pro Pro Ala Thr Thr Ser Ala Pro Ala Gly Gly Pro Thr Gln
        275                 280                 285

Thr Leu Tyr Gly Gln Cys Gly Gly Ser Gly Tyr Ser Gly Pro Thr Arg
    290                 295                 300

Cys Ala Pro Pro Ala Thr Cys Ser Thr Asn Pro Tyr Tyr Ala Gln Cys
305                 310                 315                 320

Leu Asn

<210>  13
<211>  761
<212>  PRT
<213>  Trichoderma reesei

<400>  13

Ala Phe Ser Trp Lys Asn Val Lys Leu Gly Gly Gly Gly Gly Phe Val
1               5                   10                  15

Pro Gly Ile Ile Phe His Pro Lys Thr Lys Gly Val Ala Tyr Ala Arg
            20                  25                  30

Thr Asp Ile Gly Gly Leu Tyr Arg Leu Asn Ala Asp Asp Ser Trp Thr
        35                  40                  45

Ala Val Thr Asp Gly Ile Ala Asp Asn Ala Gly Trp His Asn Trp Gly
    50                  55                  60

Ile Asp Ala Val Ala Leu Asp Pro Gln Asp Asp Gln Lys Val Tyr Ala
65                  70                  75                  80

Ala Val Gly Met Tyr Thr Asn Ser Trp Asp Pro Ser Asn Gly Ala Ile
                85                  90                  95

Ile Arg Ser Ser Asp Arg Gly Ala Thr Trp Ser Phe Thr Asn Leu Pro
            100                 105                 110

Phe Lys Val Gly Gly Asn Met Pro Gly Arg Gly Ala Gly Glu Arg Leu
        115                 120                 125

Ala Val Asp Pro Ala Asn Ser Asn Ile Ile Tyr Phe Gly Ala Arg Ser
    130                 135                 140

Gly Asn Gly Leu Trp Lys Ser Thr Asp Gly Gly Val Thr Phe Ser Lys
145                 150                 155                 160

Val Ser Ser Phe Thr Ala Thr Gly Thr Tyr Ile Pro Asp Pro Ser Asp
                165                 170                 175

Ser Asn Gly Tyr Asn Ser Asp Lys Gln Gly Leu Met Trp Val Thr Phe
            180                 185                 190

Asp Ser Thr Ser Ser Thr Thr Gly Gly Ala Thr Ser Arg Ile Phe Val
        195                 200                 205

Gly Thr Ala Asp Asn Ile Thr Ala Ser Val Tyr Val Ser Thr Asn Ala
    210                 215                 220

Gly Ser Thr Trp Ser Ala Val Pro Gly Gln Pro Gly Lys Tyr Phe Pro
225                 230                 235                 240

His Lys Ala Lys Leu Gln Pro Ala Glu Lys Ala Leu Tyr Leu Thr Tyr
                245                 250                 255

Ser Trp Trp Pro Asp Ala Gln Leu Phe Arg Ser Thr Asp Ser Gly Thr
            260                 265                 270

Thr Trp Ser Pro Ile Trp Ala Trp Ala Ser Tyr Pro Thr Glu Thr Tyr
        275                 280                 285

Tyr Tyr Ser Ile Ser Thr Pro Lys Ala Pro Trp Ile Lys Asn Asn Phe
    290                 295                 300

Ile Asp Val Thr Ser Glu Ser Pro Ser Asp Gly Leu Ile Lys Arg Leu
305                 310                 315                 320

Gly Trp Met Ile Glu Ser Leu Glu Ile Asp Pro Thr Asp Ser Asn His
                325                 330                 335

Trp Leu Tyr Gly Thr Gly Met Thr Ile Phe Gly Gly His Asp Leu Thr
            340                 345                 350

Asn Trp Asp Thr Arg His Asn Val Ser Ile Gln Ser Leu Ala Asp Gly
        355                 360                 365

Ile Glu Glu Phe Ser Val Gln Asp Leu Ala Ser Ala Pro Gly Gly Ser
    370                 375                 380

Glu Leu Leu Ala Ala Val Gly Asp Asp Asn Gly Phe Thr Phe Ala Ser
385                 390                 395                 400

Arg Asn Asp Leu Gly Thr Ser Pro Gln Thr Val Trp Ala Thr Pro Thr
                405                 410                 415

Trp Ala Thr Ser Thr Ser Val Asp Tyr Ala Gly Asn Ser Val Lys Ser
            420                 425                 430

Val Val Arg Val Gly Asn Thr Ala Gly Thr Gln Gln Val Ala Ile Ser
        435                 440                 445

Ser Asp Gly Gly Ala Thr Trp Ser Ile Asp Tyr Ala Ala Asp Thr Ser
    450                 455                 460

Met Asn Gly Gly Thr Val Ala Tyr Ser Ala Asp Gly Asp Thr Ile Leu
465                 470                 475                 480

Trp Ser Thr Ala Ser Ser Gly Val Gln Arg Ser Gln Phe Gln Gly Ser
                485                 490                 495

Phe Ala Ser Val Ser Ser Leu Pro Ala Gly Ala Val Ile Ala Ser Asp
            500                 505                 510

Lys Lys Thr Asn Ser Val Phe Tyr Ala Gly Ser Gly Ser Thr Phe Tyr
        515                 520                 525

Val Ser Lys Asp Thr Gly Ser Ser Phe Thr Arg Gly Pro Lys Leu Gly
    530                 535                 540

Ser Ala Gly Thr Ile Arg Asp Ile Ala Ala His Pro Thr Thr Ala Gly
545                 550                 555                 560

Thr Leu Tyr Val Ser Thr Asp Val Gly Ile Phe Arg Ser Thr Asp Ser
                565                 570                 575

Gly Thr Thr Phe Gly Gln Val Ser Thr Ala Leu Thr Asn Thr Tyr Gln
            580                 585                 590

Ile Ala Leu Gly Val Gly Ser Gly Ser Asn Trp Asn Leu Tyr Ala Phe
        595                 600                 605

Gly Thr Gly Pro Ser Gly Ala Arg Leu Tyr Ala Ser Gly Asp Ser Gly
    610                 615                 620

Ala Ser Trp Thr Asp Ile Gln Gly Ser Gln Gly Phe Gly Ser Ile Asp
625                 630                 635                 640

Ser Thr Lys Val Ala Gly Ser Gly Ser Thr Ala Gly Gln Val Tyr Val
                645                 650                 655

Gly Thr Asn Gly Arg Gly Val Phe Tyr Ala Gln Gly Thr Val Gly Gly
            660                 665                 670

Gly Thr Gly Gly Thr Ser Ser Ser Thr Lys Gln Ser Ser Ser Ser Thr
        675                 680                 685

Ser Ser Ala Ser Ser Ser Thr Thr Leu Arg Ser Ser Val Val Ser Thr

690                 695                 700

Thr Arg Ala Ser Thr Val Thr Ser Ser Arg Thr Ser Ser Ala Ala Gly
705                 710                 715                 720

Pro Thr Gly Ser Gly Val Ala Gly His Tyr Ala Gln Cys Gly Gly Ile
                725                 730                 735

Gly Trp Thr Gly Pro Thr Gln Cys Val Ala Pro Tyr Val Cys Gln Lys
            740                 745                 750

Gln Asn Asp Tyr Tyr Tyr Gln Cys Val
        755                 760

<210>  14
<211>  230
<212>  PRT
<213>  Trichoderma reesei

<400>  14

His Gly Gln Val Gln Asn Phe Thr Ile Asn Gly Gln Tyr Asn Gln Gly
1               5                   10                  15

Phe Ile Leu Asp Tyr Tyr Tyr Gln Lys Gln Asn Thr Gly His Phe Pro
            20                  25                  30

Asn Val Ala Gly Trp Tyr Ala Glu Asp Leu Asp Leu Gly Phe Ile Ser
        35                  40                  45

Pro Asp Gln Tyr Thr Thr Pro Asp Ile Val Cys His Lys Asn Ala Ala
    50                  55                  60

Pro Gly Ala Ile Ser Ala Thr Ala Ala Ala Gly Ser Asn Ile Val Phe
65                  70                  75                  80

Gln Trp Gly Pro Gly Val Trp Pro His Pro Tyr Gly Pro Ile Val Thr
                85                  90                  95

Tyr Val Val Glu Cys Ser Gly Ser Cys Thr Thr Val Asn Lys Asn Asn
            100                 105                 110

Leu Arg Trp Val Lys Ile Gln Glu Ala Gly Ile Asn Tyr Asn Thr Gln
        115                 120                 125

Val Trp Ala Gln Gln Asp Leu Ile Asn Gln Gly Asn Lys Trp Thr Val
    130                 135                 140

Lys Ile Pro Ser Ser Leu Arg Pro Gly Asn Tyr Val Phe Arg His Glu

145                 150                 155                 160

Leu Leu Ala Ala His Gly Ala Ser Ser Ala Asn Gly Met Gln Asn Tyr
                165                 170                 175

Pro Gln Cys Val Asn Ile Ala Val Thr Gly Ser Gly Thr Lys Ala Leu
            180                 185                 190

Pro Ala Gly Thr Pro Ala Thr Gln Leu Tyr Lys Pro Thr Asp Pro Gly
        195                 200                 205

Ile Leu Phe Asn Pro Tyr Thr Thr Ile Thr Ser Tyr Thr Ile Pro Gly
    210                 215                 220

Pro Ala Leu Trp Gln Gly
225                 230

<210>  15
<211>  419
<212>  PRT
<213>  Trichoderma reesei

<400>  15

Gly Lys Ile Lys Tyr Leu Gly Val Ala Ile Pro Gly Ile Asp Phe Gly
1               5                   10                  15

Cys Asp Ile Asp Gly Ser Cys Pro Thr Asp Thr Ser Ser Val Pro Leu
            20                  25                  30

Leu Ser Tyr Lys Gly Gly Asp Gly Ala Gly Gln Met Lys His Phe Ala
        35                  40                  45

Glu Asp Asp Gly Leu Asn Val Phe Arg Ile Ser Ala Thr Trp Gln Phe
    50                  55                  60

Val Leu Asn Asn Thr Val Asp Gly Lys Leu Asp Glu Leu Asn Trp Gly
65                  70                  75                  80

Ser Tyr Asn Lys Val Val Asn Ala Cys Leu Glu Thr Gly Ala Tyr Cys
                85                  90                  95

Met Ile Asp Met His Asn Phe Ala Arg Tyr Asn Gly Gly Ile Ile Gly
            100                 105                 110

Gln Gly Gly Val Ser Asp Asp Ile Phe Val Asp Leu Trp Val Gln Ile
        115                 120                 125

Ala Lys Tyr Tyr Glu Asp Asn Asp Lys Ile Ile Phe Gly Leu Met Asn

130                 135                 140

Glu Pro His Asp Leu Asp Ile Glu Ile Trp Ala Gln Thr Cys Gln Lys
145                 150                 155                 160

Val Val Thr Ala Ile Arg Lys Ala Gly Ala Thr Ser Gln Met Ile Leu
                165                 170                 175

Leu Pro Gly Thr Asn Phe Ala Ser Val Glu Thr Tyr Val Ser Thr Gly
            180                 185                 190

Ser Ala Glu Ala Leu Gly Lys Ile Thr Asn Pro Asp Gly Ser Thr Asp
        195                 200                 205

Leu Leu Tyr Phe Asp Val His Lys Tyr Leu Asp Ile Asn Asn Ser Gly
    210                 215                 220

Ser His Ala Glu Cys Thr Thr Asp Asn Val Asp Ala Phe Asn Asp Phe
225                 230                 235                 240

Ala Asp Trp Leu Arg Gln Asn Lys Arg Gln Ala Ile Ile Ser Glu Thr
                245                 250                 255

Gly Ala Ser Met Glu Pro Ser Cys Met Thr Ala Phe Cys Ala Gln Asn
            260                 265                 270

Lys Ala Ile Ser Glu Asn Ser Asp Val Tyr Ile Gly Phe Val Gly Trp
        275                 280                 285

Gly Ala Gly Ser Phe Asp Thr Ser Tyr Ile Leu Thr Leu Thr Pro Leu
    290                 295                 300

Gly Lys Pro Gly Asn Tyr Thr Asp Asn Lys Leu Met Asn Glu Cys Ile
305                 310                 315                 320

Leu Asp Gln Phe Thr Leu Asp Glu Lys Tyr Arg Pro Thr Pro Thr Ser
                325                 330                 335

Ile Ser Thr Ala Ala Glu Glu Thr Ala Thr Ala Thr Ala Thr Ser Asp
            340                 345                 350

Gly Asp Ala Pro Ser Thr Thr Lys Pro Ile Phe Arg Glu Glu Thr Ala
        355                 360                 365

Ser Pro Thr Pro Asn Ala Val Thr Lys Pro Ser Pro Asp Thr Ser Asp
    370                 375                 380

Ser Ser Asp Asp Asp Lys Asp Ser Ala Ala Ser Met Ser Ala Gln Gly
385                 390                 395                 400

Leu Thr Gly Thr Val Leu Phe Thr Val Ala Ala Leu Gly Tyr Met Leu
                405                 410                 415

Val Ala Phe

<210>  16
<211>  499
<212>  PRT
<213>  Bacillus subtilis

<400>  16

Met Lys Arg Ser Ile Ser Ile Phe Ile Thr Cys Leu Leu Ile Thr Leu
1               5                   10                  15

Leu Thr Met Gly Gly Met Ile Ala Ser Pro Ala Ser Ala Ala Gly Thr
            20                  25                  30

Lys Thr Pro Val Ala Lys Asn Gly Gln Leu Ser Ile Lys Gly Thr Gln
        35                  40                  45

Leu Val Asn Arg Asp Gly Lys Ala Val Gln Leu Lys Gly Ile Ser Ser
    50                  55                  60

His Gly Leu Gln Trp Tyr Gly Glu Tyr Val Asn Lys Asp Ser Leu Lys
65                  70                  75                  80

Trp Leu Arg Asp Asp Trp Gly Ile Thr Val Phe Arg Ala Ala Met Tyr
                85                  90                  95

Thr Ala Asp Gly Gly Tyr Ile Asp Asn Pro Ser Val Lys Asn Lys Val
            100                 105                 110

Lys Glu Ala Val Glu Ala Ala Lys Glu Leu Gly Ile Tyr Val Ile Ile
        115                 120                 125

Asp Trp His Ile Leu Asn Asp Gly Asn Pro Asn Gln Asn Lys Glu Lys
    130                 135                 140

Ala Lys Glu Phe Phe Lys Glu Met Ser Ser Leu Tyr Gly Asn Thr Pro
145                 150                 155                 160

Asn Val Ile Tyr Glu Ile Ala Asn Glu Pro Asn Gly Asp Val Asn Trp
                165                 170                 175

Lys Arg Asp Ile Lys Pro Tyr Ala Glu Glu Val Ile Ser Val Ile Arg
            180                 185                 190

Lys Asn Asp Pro Asp Asn Ile Ile Ile Val Gly Thr Gly Thr Trp Ser
        195                 200                 205

Gln Asp Val Asn Asp Ala Ala Asp Asp Gln Leu Lys Asp Ala Asn Val
    210                 215                 220

Met Tyr Ala Leu His Phe Tyr Ala Gly Thr His Gly Gln Phe Leu Arg
225                 230                 235                 240

Asp Lys Ala Asn Tyr Ala Leu Ser Lys Gly Ala Pro Ile Phe Val Thr
                245                 250                 255

Glu Trp Gly Thr Ser Asp Ala Ser Gly Asn Gly Gly Val Phe Leu Asp
            260                 265                 270

Gln Ser Arg Glu Trp Leu Lys Tyr Leu Asp Ser Lys Thr Ile Ser Trp
        275                 280                 285

Val Asn Trp Asn Leu Ser Asp Lys Gln Glu Ser Ser Ser Ala Leu Lys
    290                 295                 300

Pro Gly Ala Ser Lys Thr Gly Gly Trp Arg Leu Ser Asp Leu Ser Ala
305                 310                 315                 320

Ser Gly Thr Phe Val Arg Glu Asn Ile Leu Gly Thr Lys Asp Ser Thr
                325                 330                 335

Lys Asp Ile Pro Glu Thr Pro Ser Lys Asp Lys Pro Thr Gln Glu Asn
            340                 345                 350

Gly Ile Ser Val Gln Tyr Arg Ala Gly Asp Gly Ser Met Asn Ser Asn
        355                 360                 365

Gln Ile Arg Pro Gln Leu Gln Ile Lys Asn Asn Gly Asn Thr Thr Val
    370                 375                 380

Asp Leu Lys Asp Val Thr Ala Arg Tyr Trp Tyr Lys Ala Lys Asn Lys
385                 390                 395                 400

Gly Gln Asn Phe Asp Cys Asp Tyr Ala Gln Ile Gly Cys Gly Asn Val
                405                 410                 415

Thr His Lys Phe Val Thr Leu His Lys Pro Lys Gln Gly Ala Asp Thr
            420                 425                 430

Tyr Leu Glu Leu Gly Phe Lys Asn Gly Thr Leu Ala Pro Gly Ala Ser
        435                 440                 445

Thr Gly Asn Ile Gln Leu Arg Leu His Asn Asp Asp Trp Ser Asn Tyr
    450                 455                 460

Ala Gln Ser Gly Asp Tyr Ser Phe Phe Lys Ser Asn Thr Phe Lys Thr
465                 470                 475                 480

Thr Lys Lys Ile Thr Leu Tyr Asp Gln Gly Lys Leu Ile Trp Gly Thr
                485                 490                 495

Glu Pro Asn

<210>  17
<211>  185
<212>  PRT
<213>  Artificial sequence

<220>
<223>  Bacillus subtilis xylanase variant

<400>  17

Ala Ser Thr Asp Tyr Trp Gln Asn Trp Thr Phe Gly Gly Gly Ile Val
1               5                   10                  15

Asn Ala Val Asn Gly Ser Gly Gly Asn Tyr Ser Val Asn Trp Ser Asn
            20                  25                  30

Thr Gly Asn Phe Val Val Gly Lys Gly Trp Thr Thr Gly Ser Pro Phe
        35                  40                  45

Arg Thr Ile Asn Tyr Asn Ala Gly Val Trp Ala Pro Asn Gly Asn Gly
    50                  55                  60

Tyr Leu Thr Leu Tyr Gly Trp Thr Arg Ser Pro Leu Ile Glu Tyr Tyr
65                  70                  75                  80

Val Val Asp Ser Trp Gly Thr Tyr Arg Pro Thr Gly Thr Tyr Lys Gly
                85                  90                  95

Thr Val Lys Ser Asp Gly Gly Thr Tyr Asp Ile Tyr Thr Thr Thr Arg
            100                 105                 110

Tyr Asn Ala Pro Ser Ile Asp Gly Asp Asp Thr Thr Phe Thr Gln Tyr
        115                 120                 125

Trp Ser Val Arg Gln Ser Lys Arg Pro Thr Gly Ser Asn Ala Thr Ile
    130                 135                 140

Thr Phe Ser Asn His Val Asn Ala Trp Lys Ser His Gly Met Asn Leu
145                 150                 155                 160

Gly Ser Asn Trp Ala Tyr Gln Val Met Ala Thr Glu Gly Tyr Gln Ser
                165                 170                 175

Ser Gly Ser Ser Asn Val Thr Val Trp
            180                 185

<210>  18
<211>  185
<212>  PRT
<213>  Artificial sequence

<220>
<223>  Bacillus subtilis xylanase variant

<400>  18

Ala Ser Thr Asp Tyr Trp Gln Asn Trp Thr Asp Gly Tyr Gly Ile Val
1               5                   10                  15

Asn Ala Val Asn Gly Ser Gly Gly Asn Tyr Ser Val Asn Trp Ser Asn
            20                  25                  30

Thr Gly Asn Phe Val Val Gly Lys Gly Trp Thr Thr Gly Ser Pro Phe
        35                  40                  45

Arg Thr Ile Asn Tyr Asn Ala Gly Val Trp Ala Pro Asn Gly Asn Gly
    50                  55                  60

Tyr Leu Thr Leu Tyr Gly Trp Thr Arg Ser Pro Leu Ile Glu Tyr Tyr
65                  70                  75                  80

Val Val Asp Ser Trp Gly Thr Tyr Arg Pro Thr Gly Thr Tyr Lys Gly
                85                  90                  95

Thr Val Tyr Ser Asp Gly Gly Trp Tyr Asp Ile Tyr Thr Ala Thr Arg
            100                 105                 110

Asp Asn Ala Pro Ser Ile Asp Gly Asp Phe Thr Thr Phe Thr Gln Tyr
        115                 120                 125

Trp Ser Val Arg Gln Ser Lys Arg Pro Thr Gly Ser Asn Ala Thr Ile
    130                 135                 140

Thr Phe Ser Asn His Val Asn Ala Trp Arg Ser His Gly Met Asp Leu

145                 150                 155                 160

Gly Ser Asn Trp Ala Tyr Gln Val Met Ala Thr Glu Gly Tyr Leu Ser
                165                 170                 175

Ser Gly Ser Ser Asn Val Thr Val Trp
            180                 185